# EUROPEAN PATENT SPECIFICATION

(11) **EP 2 387 566 B1**
(45) Date of publication and mention of the grant of the patent: **07.05.2014**
(21) Application number: 10700989.6
(22) Date of filing: 14.01.2010
(51) Int. Cl.: C07D 239/42

(54) **PROCESS FOR THE PREPARATION OF ROSUVASTATIN**
VERFAHREN ZUR HERSTELLUNG VON ROSUVASTATIN
PROCÉDÉ POUR LA PRÉPARATION DE ROSUVASTATIN

(30) Priority: 14.01.2009 SI 200900012; 31.07.2009 SI 200900217
(43) Date of publication of application: 23.11.2011
(60) Divisional application: 14162586.3
(73) Proprietor: KRKA, tovarna zdravil, d.d., Novo mesto, 8501 Novo mesto (SI)
(72) Inventor: BENKIC, Primoz, 1000 Ljubljana (SI); BEVK, David, 8000 Novo mesto (SI); LENARSIC, Roman, 1000 Ljubljana (SI); ZUPANCIC, Silvo, 8000 Novo mesto (SI); VAJS, Anamarija, 8000 Novo mesto (SI); JAKSE, David, 8323 Ursna sela (SI)
(74) Representative: UEXKÜLL & STOLBERG
(86) International application number: PCT/EP2010/050425
(87) International publication number: WO 2010/081861

(56) References cited:
- WO-A1-01/60804
- WO-A1-2005/023779
- WO-A1-2005/051921
- WO-A1-2005/077916
- WO-A1-2006/079611
- WO-A1-2006/136407
- WO-A2-2006/136408
- WO-A2-2007/125547
- WO-A2-2008/044243

## Description

The invention relates to salts of HMG-CoA reductase inhibitors and processes for preparing same as well as processes for preparing pharmaceutically acceptable salts of HMG-CoA reductase inhibitors.

### Background of the invention

The compound of the formula IA below is rosuvastatin, the chemical name of which is (E)-7-{4-(4-fluorophenyl)-6-isopropyl-2-[methyl(methylsulfonyl)amino]-pyrimidin-5-yl}-(3R,5S)-3,5-dihydroxy-hept-6-enoic acid

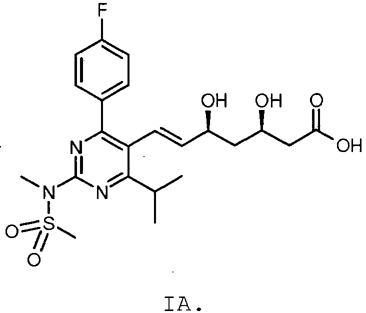

Rosuvastatin as well as its sodium and calcium salt are disclosed in EP 0 521 471 A1 as 3-hydroxy-3-methylglutaryl-coenzyme A (HMG-CoA) reductase inhibitors, which can be used in the treatment of hypercholesterolemia, hyperlipoproteinemia and atherosclerosis.

WO 00/49014 A1 discloses a process for the preparation of a rosuvastatin precursor involving the synthesis of the rosuvastatin double bond by reacting a pyrimidinylmethylphosphine oxide with a bis-protected 6-oxo-3,5-dihydroxy-hexanoic acid at a temperature in the range of from -90 °C to -20 °C.

WO 2005/054207 A1 discloses a process for preparing pyrimidine derivatives such as rosuvastatin, wherein the rosuvastatin double bond is synthesized by reaction of a pyrimidinylmethylphosphine with a 6-oxo-3,5-dihydroxy-hexanoic acid derivative.

CN 1821242 A discloses a process for the preparation of rosuvastatin involving a Horner-Wadsworth-Emmons reaction of a pyrimidine derivative with a phosphonate reagent.

It has been found that the prior art processes for the preparation of rosuvastatin suffer from a number of drawbacks, such as the need for protecting groups or the number of reaction steps. Thus, there remains a need for a process for preparing rosuvastatin which overcomes the problems of the prior art and is better suited for application on an industrial scale.

### Brief description of the drawings

Fig. 1 shows an XRPD pattern of rosuvastatin 1-[(4-chlorophenyl)-phenyl-methyl]piperazinium salt as obtained in Example 2-1.
Fig. 2 shows an FT-IR spectrum of rosuvastatin 1-[(4-chlorophenyl)-phenyl-methyl]piperazinium salt as obtained in Example 2-1.
Fig. 3 shows an XRPD pattern of rosuvastatin (-)-1-[(4-chlorophenyl)-phenyl-methyl]piperazinium salt as obtained in Example 2-2.
Fig. 4 shows an FT-IR spectrum of rosuvastatin (-)-1-[(4-chlorophenyl)-phenyl-methyl]piperazinium salt as obtained in Example 2-2.
Fig. 5 shows an XRPD pattern of amorphous rosuvastatin calcium as obtained in Example 3-4.
Fig. 6 shows a microphotograph of the amorphous rosuvastatin calcium obtained according to Example 4, Experiment A.
Fig. 7 shows a microphotograph of amorphous rosuvastatin calcium obtained according to Example 4, Experiment B.
Fig. 8 shows an XRPD pattern of crystalline rosuvastatin calcium trihydrate as obtained in Example 5.
Fig. 9 shows an FT-IR spectrum of crystalline rosuvastatin calcium trihydrate as obtained in Example 5.
Fig. 10 shows an XRPD pattern of crystalline anhydrous rosuvastatin calcium as obtained in Example 6.

### Description of the invention

An exemplary process for the preparation of a compound of formula II 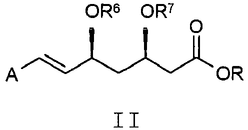 comprises the step of
reacting a compound of formula III 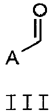 with a compound of formula IV 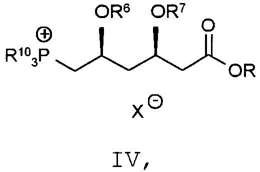 wherein
A is selected from the group consisting of 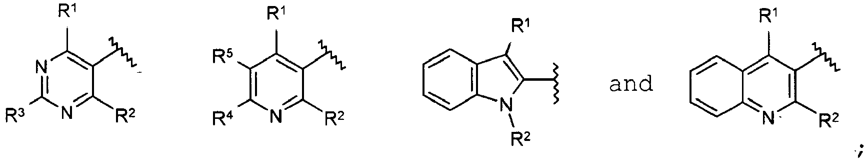 R is selected from the group consisting of H and (C₁-C₁₂)alkyl, preferably methyl and tert-butyl;
R¹, R², R⁴ and R⁵ are independently selected from the group consisting of H, (C₁-C₁₂)alkyl, (C₃-C₆)cycloalkyl, aryl and aryl(C₁-C₁₂)alkyl, wherein each of said alkyl and aryl may have one or more substituents independently selected from halogen, (C₁-C₁₂)alkyl, (C₁-C₁₂)alkoxy, aryl, aryl (C₁-C₁₂)alkyl and aryl(C₁-C₁₂)alkoxy;
R³ is selected from the group consisting of -S-R¹¹, -O-R¹¹, -SO₂-R¹¹, -N(R¹²)-R¹¹, -N(R¹²)-CO-R¹¹, -N(R¹²)-SO₂-R¹¹ and -N(R¹²)-NH-SO₂-R¹¹;
R⁶ and R⁷ are independently H or a hydroxyl protecting group, or R⁶ and R⁷ together with the oxygen atoms to which they are attached form a group of formula -O-(CR⁸R⁹)-O-;
R⁸ and R⁹ are independently selected from the group consisting of H, (C₁-C₁₂)alkyl, aryl and aryl(C₁-C₁₂)alkyl;
R¹⁰ is selected from (C₁-C₁₂)alkyl, aryl or aryl(C₁-C₁₂)alkyl, each of which may have one or more substituents selected from halogen, (C₁-C₁₂)alkyl, aryl and aryl(C₁-C₁₂)alkyl;
R¹¹ is selected from the group consisting of (C₁-C₁₂)alkyl, aryl and aryl(C₁-C₁₂)alkyl, each of which may have one or more substituents independently selected from halogen, (C₁-C₁₂)alkyl, aryl and aryl(C₁-C₁₂)alkyl;
R¹² is selected from the group consisting of H, (C₁-C₁₂)alkyl, aryl and aryl(C₁-C₁₂)alkyl; and
X is selected from the group consisting of Cl, Br, I, F₃CSO₂O, H₃CSO₂O and (4-methylphenyl)-SO₂O.

This process provides a number of advantages. In particular, it has been found that the intermediates of formulae III and IV are much better accessible on an industrial scale than intermediates used in prior processes. Surprisingly, compounds of formula IV can be easily prepared in high yields. Moreover, these compounds have been found to be sufficiently stable for purification, e.g. by way of re-crystallization, which in turn allows for the preparation of compounds of formula II in better yields and purity. In addition, the process dispenses with the need associated with many prior art processes to prepare problematic intermediates such as 6-oxo-3,5-dihydroxy-hexanoic acid derivatives.

According to a preferred embodiment,
R is H or (C₁-C₁₂)alkyl, preferably methyl and tert-butyl;
R¹ is aryl, which may have one or more substituents independently selected from halogen, (C₁-C₁₂)alkyl, (C₁-C₁₂)alkoxy, aryl, aryl(C₁-C₁₂)alkyl and aryl (C₁-C₁₂)alkoxy;
R² is selected from the group consisting of (C₁-C₆)alkyl and (C₃-C₆)cycloalkyl;
R³ is -N(R¹²)-SO₂-R¹¹, wherein R¹¹ and R¹² are independently selected from (C₁-C₆)alkyl;
R⁴ is selected from the group consisting of (C₁-C₆)alkyl; and
R⁵ is selected from the group consisting of (C₁-C₆)alkoxy(C₁-C₆)alkyl.

According to a particularly preferred embodiment,
R is H or (C₁-C₁₂)alkyl, preferably methyl and tert-butyl;
R¹ is 4-fluorophenyl;
R² is isopropyl or cyclopropyl;
R³ is -N(CH₃)-SO₂-CH₃;
R⁴ is isopropyl; and
R⁵ is methoxymethyl.

Most preferably, A is selected from the group consisting of

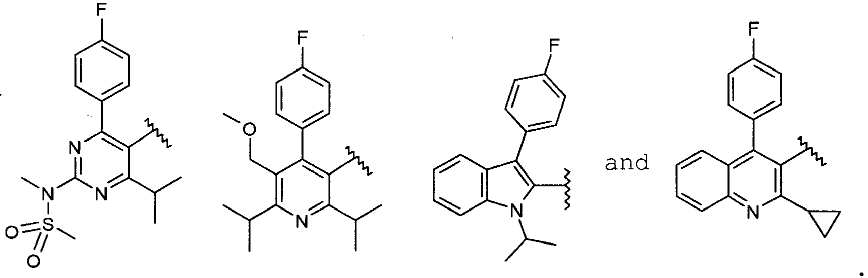

These groups A correspond to the known HMG-CoA reductase inhibitors rosuvastatin, cerivastatin, fluvastatin and pitavastatin, respectively.

R⁶ and R⁷ may independently be a suitable hydroxyl protecting group, preferably a hydrolyzable protecting group. Examples of suitable hydroxyl protecting groups include silyl protecting groups such as tert-butyldimethylsilyl, trimethylsilyl or triisopropylsilyl.

According to a preferred embodiment, R⁶ and R⁷ together with the oxygen atoms to which they are attached form a group of formula -O-(CR⁸R⁹)-O-, wherein R⁸ and R⁹ are independently selected from the group consisting of H, (C₁-C₁₂)alkyl, aryl and aryl(C₁-C₁₂)alkyl. In one embodiment R⁸ and R⁹ are each methyl. According to another embodiment, R⁸ is aryl and R⁹ is H. It is particularly preferred that R⁸ is phenyl and R⁹ is H. The benzylidene protecting group has been found to provide for a particularly high stability of the intermediates used in the process. This improved stability allows for easier purification of the intermediates, e.g. by way of column chromatography, and improved purity of final product.

According to another preferred embodiment, R⁶ and R⁷ are both H. Surprisingly, it has been found that the process allows to synthesize the double bond of the compound of formula II without the need for protecting the hydroxyl groups. Thus, the process according to the reaction can be performed using substantially less reaction steps. Moreover, the possibility to dispense with protection of the hydroxyl groups also removes the need for the subsequent step of removing such protecting groups, e.g. using acid catalysis, which step may result in formation of lactone by-product often observed in prior art processes.

R is preferably selected from the group consisting of methyl, ethyl, propyl, isopropyl, butyl, isobutyl and tert-butyl. Most preferably, R is methyl, ethyl, isopropyl or tert-butyl.

R¹⁰ is preferably selected from unsubstituted or substituted aryl, most preferably phenyl.

It is further preferred that X is selected from the group consisting of I, F₃CSO₂O, H₃CSO₂O and (4-methylphenyl)SO₂O. Most preferably, X is I.

The reaction may be carried out in a suitable solvent. Examples of suitable solvents include aromatic hydrocarbons such as benzene, toluene, dimethylformamide, dimethylacetamide, dimethylsulfoxide, acetonitrile, diethylether, tetrahydrofuran and mixtures thereof. Preferably, the solvent is selected from aromatic hydrocarbons and mixtures thereof, most preferably toluene.

The reaction may be performed at a temperature in the range of from 0 °C to the reflux temperature of the solvent. Preferably, the reaction is performed at a temperature in the range of from 0 °C to 120 °C, more preferably 15 °C to 80 °C, still more preferably 20 °C to 40 °C, most preferably 20 °C to 35 °C.

The reaction is performed in the presence of a base. Examples of suitable bases include alkali or alkaline earth metal carbonates, hydroxides and alkoxides as well as primary, secondary and tertiary alkylamines. Preferred bases are Na₂CO₃, K₂CO₃, KotBu and Et₃N.

Preferably, the base is used in an amount of from 1 to 5 equivalents, more preferably 1.5 to 4 equivalents, most preferably 2 to 3 equivalents with respect to the compound of formula IV.

The compound of formula III is generally used in an at least equimolar amount with respect to the compound of formula IV. Preferably, the compound of formula III is used in an amount of 1 to 2 equivalents, more preferably 1.1 to 1.5 equivalents, most preferably 1.2 to 1.3 equivalents with respect to the compound of formula IV.

The process according may further comprise changing at least one of groups R⁶ and R⁷ in the compound of formula II to obtain a compound of formula II wherein R⁶ and R⁷ are both H and which is also referred to as compound I.

According to a preferred embodiment, the compound of formula IV is obtained by converting a compound of formula V 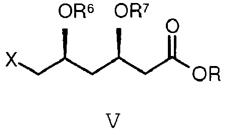 to the compound of formula IV using a phosphine reagent of formula PR¹⁰₃.

The conversion may be carried out in a suitable solvent, such as acetonitrile, at a temperature in the range of from 20 °C to the reflux temperature of the solvent. Preferably, the resulting compound of formula IV is collected by filtration. The compound of formula IV may be further purified by re-crystallization.

The process may further comprise changing at least one of groups R⁶ and R⁷ in the compound of formula V prior to converting said compound to the compound of formula IV.

According to a further preferred embodiment, the compound of formula V is obtained by converting a compound of formula VI 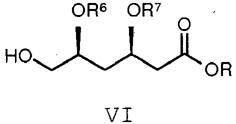 to the compound of formula V.

This conversion can be achieved using a reagent generally used for converting an alcoholic hydroxyl group to group X. Examples of suitable reagents include PCl₃, PBr₃, PPh₃/CBr₄, PPh₃/I₂, F₃CSO₂Cl, H₃CSO₂Cl and (4-methylphenyl) -SO₂Cl. Typically, the compound of formula VI is dissolved in a solvent such as e.g. CH₂Cl₂, toluene or hexane, and a reagent suitable to introduce the group X is added in the presence of a base such as e.g. DMAP, Et₃N or imidazole. The reaction may be performed at a temperature in the range of from 0 °C to room temperature.

The process may further comprise changing at least one of groups R⁶, R⁷ and R in the compound of formula VI prior to converting said compound to the compound of formula V.

Where the process according comprises one or more steps of changing one or more of groups R⁶, R⁷ and R, such steps can be performed using conditions generally known in the art for removing or introducing the respective protecting groups.

For example, an acetonide protecting group (R⁸ = R⁹ = Me) may be removed by treatment with an acid, such as 80 % aqueous acetic acid, at room temperature. A benzylidene protecting group (R⁸ = phenyl, R⁹ = H) may be removed by treatment with aqueous HCl in a solvent, such as methanol, ethanol or acetonitrile, at room temperature or elevated temperature. Introduction of a benzylidene protecting group may be achieved by treatment with benzaldehyde dimethylacetal and pyridinium p-toluenesulfonate in a solvent such as dichloromethane at room temperature. Removal of silyl protecting groups (R⁶ and/or R⁷ = Si(alkyl)₃) may be achieved by treatment with TBAF or HF in a solvent such as dichloromethane or THF at room temperature or elevated temperature. A tert-butylester group (R = t-butyl) may be exchanged for a different ester group by heating to reflux temperature in the respective alcohol in the presence of an acidic or alkaline catalyst.

A particular process for the preparation of a compound of formula II 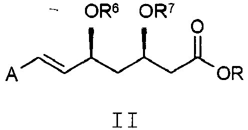 comprises the steps of
(a) converting a compound of formula VI 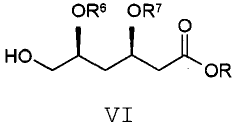 to a compound of formula V 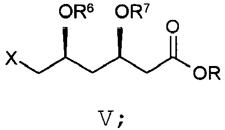
(b) optionally converting the compound of formula V to a compound of formula V differing in at least one of the groups R⁶ and R⁷;
(c) converting the compound of formula V to a compound of formula IV 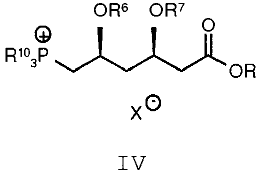 using a phosphine reagent of formula PR¹⁰₃;
(d) reacting the compound of formula IV with a compound of formula III 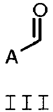 to give the compound of formula II

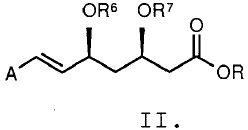

Starting compounds of formulae III and VI can be prepared according to processes generally known in the art. For example, processes for the preparation of compounds of formula III are disclosed in EP 0 521 471, WO 2007/074391, WO 2006/128954, WO 2006/017357 and WO 2003/097614. Exemplary processes for the preparation of compounds of formula VI are disclosed in EP 0 464 817, EP 4 694 801 and US 5,278,313.

An exemplary process for the preparation of a compound of formula I 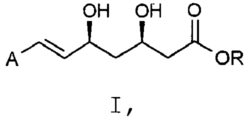 or a pharmaceutically acceptable salt or solvate thereof, comprises the steps of:
(a) preparing the compound of formula II according to the process described above, and
(b) converting the compound of formula II into the compound of formula I or the pharmaceutically acceptable salt or solvate thereof.

Preferably, the compound of formula I is rosuvastatin of formula IA

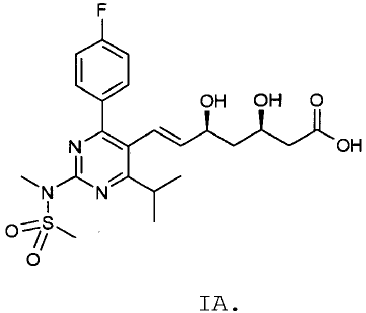

Moreover, step (b) may be achieved by base catalyzed ester hydrolysis of the ester moiety (-COOR) of the compound of formula II. Preferably, hydrolysis is achieved by treating the compound of formula II with a base, such as an alkaline hydroxide, e.g. 0.1 M NaOH, in a suitable solvent, such as ethanol. Typically, the compound of formula I is thus obtained as a respective salt. For instance, where NaOH is used in step (b), the compound of formula I is obtained as a sodium salt thereof. According to another embodiment, hydrolysis of the ester moiety of the compound of formula II can be achieved by treating the compound of formula II with an organic amine to obtain an organic ammonium salt as described below.

Optionally step (b) further comprises converting the compound of formula II to the respective compound of formula II wherein R⁶ and R⁷ are both H prior to the hydrolysis step.

The process may further comprise transforming the compound of formula I or the salt or solvate thereof to a pharmaceutically acceptable salt or solvate of the compound of the compound of formula I. For example, a sodium salt obtained by NaOH catalyzed hydrolysis of the compound of formula II may be converted to the calcium salt of the compound of formula I by reacting an aqueous solution of the sodium salt with 1 M CaCl₂ at room temperature and collecting the precipitated calcium salt of the compound of formula I.

The purity of compound II obtained by the above process is typically at least 80 %, particularly 90 % or more.

General scheme for a process for the preparation of a compound of formula I:

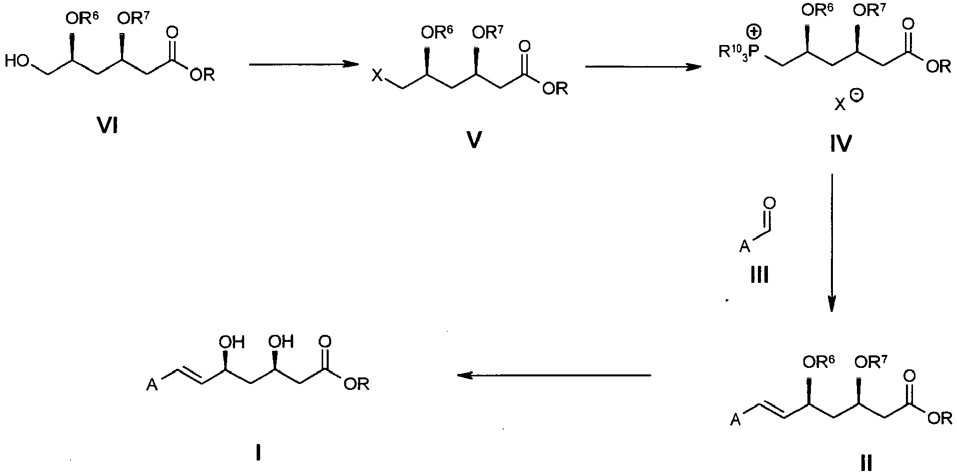

An exemplary process for the preparation of a pharmaceutical formulation comprising a compound of formula I, or a pharmaceutically acceptable salt or solvate thereof, in combination with at least one pharmaceutically acceptable carrier, includes the step of preparing the compound of formula I or the pharmaceutically acceptable salt or solvate thereof by the process described above. Preferably, the compound of formula I is selected from the group consisting of rosuvastatin, cerivastatin, fluvastatin and pitavastatin. Most preferably, the pharmaceutically acceptable salt is the sodium or calcium salt of rosuvastatin, used in the final oral dosage form as e.g. described in WO 2009/156173.

Also disclosed are intermediates which are suitable for use in the processes described above.

In one aspect there is disclosed a compound selected from the group consisting of 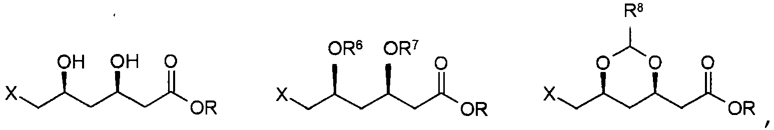 wherein:
R is selected from H and (C₁-C₁₂)alkyl, more preferably from the group consisting of methyl, ethyl, isopropyl, and tert-butyl;
R⁶ and R⁷ are independently a hydroxyl protecting group;
R⁸ is selected from the group consisting of (C₁-C₁₂)alkyl, aryl and aryl(C₁-C₁₂)alkyl, more preferably the group consisting of aryl and aryl(C₁-C₁₂)alkyl, most preferably phenyl;
X is selected from the group consisting of Cl, Br, I, F₃CSO₂O, H₃CSO₂O and (4-methylphenyl)-SO₂O, more preferably from the group consisting of I, F₃CSO₂O, H₃CSO₂O and (4-methylphenyl)-SO₂O, most preferably I.

In another aspect there is disclosed a compound selected from the group consisting of 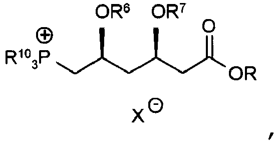 wherein:
R is selected from H and (C₁-C₁₂)alkyl, more preferably from the group consisting of methyl, ethyl, isopropyl, and tert-butyl;
R⁶ and R⁷ are independently H or a hydroxyl protecting group, or R⁶ and R⁷ together with the oxygen atoms to which they are attached form a group of formula -O-(CR⁸R⁹)-O-;
R⁸ and R⁹ are independently selected from the group consisting of H, (C₁-C₁₂)alkyl, aryl and aryl(C₁-C₁₂)alkyl;
R¹⁰ is selected from (C₁-C₁₂)alkyl, aryl or aryl(C₁-C₁₂)alkyl, each of which may have one or more substituents selected from halogen, (C₁-C₁₂)alkyl, aryl and aryl(C₁-C₁₂)alkyl;
X is selected from the group consisting of Cl, Br, I, F₃CSO₂O, H₃CSO₂O and (4-methylphenyl)-SO₂O, more preferably from the group consisting of I, F₃CSO₂O, H₃CSO₂O and (4-methylphenyl)-SO₂O, most preferably I;

In yet another aspect there is disclosed a compound selected from the group consisting of 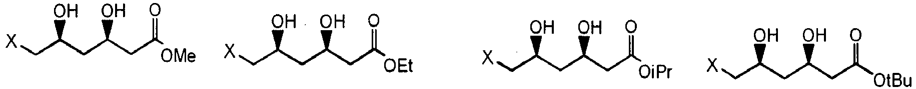 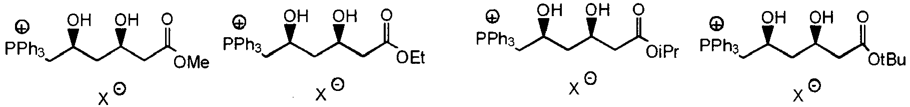 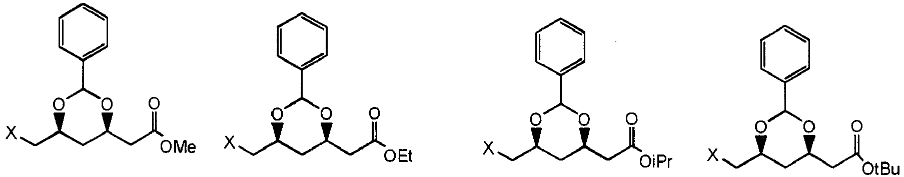 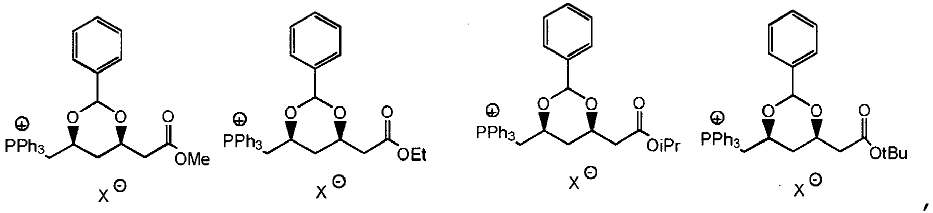 wherein X is selected from the group consisting of Cl, Br, I, F₃CSO₂O, H₃CSO₂O and (4-methylphenyl)-SO₂O, more preferably from the group consisting of I, F₃CSO₂O, H₃CSO₂O and (4-methylphenyl)-SO₂O, most preferably I.

Also disclosed is a use of any one of the compounds described immediately above for the preparation of a compound of formula II or for the preparation of the compound of formula I or pharmaceutically acceptable salts or solvates thereof.

The invention provides novel salts of rosuvastatin of formula IA and processes using such salts to prepare rosuvastatin of formula IA or salts thereof of high purity.

Thus, the invention relates to a salt of formula VII 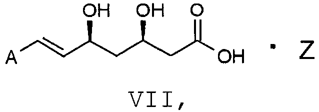 wherein:
A is 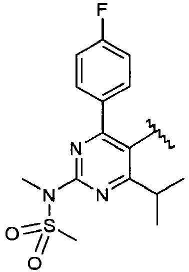 and
Z is a 4-(C₁-C₁₂-alkyl)piperazine wherein the C₁-C₁₂-alkyl group comprises at least one further substituent independently selected from the group consisting of unsubstituted or substituted aryl, heteroaryl, (C₃-C₆)cycloalkyl and (C₃-C₆)heterocycloalkyl. Suitable substituents of said aryl, heteroaryl, cycloalkyl, and heterocycloalkyl groups include halogen, (C₁-C₆)alkyl, (C₁-C₆)alkoxy, CH₂F, CHF₂ and CF₃.

It is preferred that Z is an amine of formula Z¹ 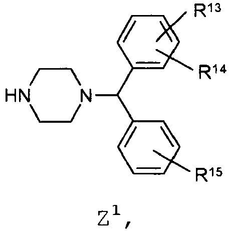 wherein R¹³, R¹⁴ and R¹⁵ are independently selected from H, halogen, (C₁-C₆)alkyl, (C₁-C₆)alkoxy, CH₂F, CHF₂ and CF₃, wherein halogen is independently selected from F, Cl, Br and I.

According to one embodiment of Z¹,
R¹³ is selected from 4-halogen, 4-(C₁-C₆)alkyl, 4-(C₁-C₆)alkoxy, 4-CH₂F, 4-CHF₂ and 4-CF₃;
R¹⁴ is selected from H, 3- or 5-halogen, 3- or 5-(C₁-C₆)alkyl, 3- or 5-(C₁-C₆)alkoxy, 3- or 5-CH₂F, 3- or 5-CHF₂ and 3- or 5-CF₃; and
R¹⁵ is H.

According to another embodiment of Z¹,
R¹³ is selected from 3-halogen, 3-(C₁-C₆)alkyl, 3-(C₁-C₆)alkoxy, 3-CH₂F, 3-CHF₂ and 3-CF₃;
R¹⁴ is selected from 5-halogen, 5-(C₁-C₆)alkyl, 5-(C₁-C₆)alkoxy, 5-CH₂F, 5-CHF₂ and 5-CF₃; and
R¹⁵ is H.

According to a preferred embodiment, Z¹ is selected from the group consisting of
1-((diphenylmethyl)piperazine;
1-[((4-chlorophenyl)-phenyl-methyl]piperazine;
1-[((4-methoxyphenyl)-phenyl-methyl]piperazine;
1-[((3,5-dichlorophenyl)-phenyl-methyl]piperazine;
1-[((3,4-dichlorophenyl)-phenyl-methyl]piperazine;
1-[((4-fluorophenyl)-phenyl-methyl]piperazine;
1-[((4-methylphenyl)-phenyl-methyl]piperazine; and
1-[((4-trifluoromethylphenyl)-phenyl-methyl]piperazine.

Amines Z may contain asymmetric or chiral centers and therefore exist in different stereoisomeric forms, and all of these forms as well as mixtures thereof, including racemic mixtures, are contemplated herein. In particular, where the two phenyl groups of the amine Z¹ are differently substituted, amine Z¹ exists in racemic form (±)-Z¹ and enantiomeric forms (-)-Z¹ and (+)-Z¹, wherein (-)-Z¹ is preferred. Thus, according to a further preferred embodiment, Z is selected from the group consisting of
(-)-1-[((4-chlorophenyl)-phenyl-methyl]piperazine;
(-)-1-[((4-methoxyphenyl)-phenyl-methyl]piperazine;
(-)-1-[((3,5-dichlorophenyl)-phenyl-methyl]piperazine;
(-)-1-[((3,4-dichlorophenyl)-phenyl-methyl]piperazine;
(-)-1-[((4-fluorophenyl)-phenyl-methyl]piperazine;
(-)-1-[((4-methylphenyl)-phenyl-methyl]piperazine; and
(-)-1-[((4-trifluoromethylphenyl)-phenyl-methyl]piperazine.

According to one particularly preferred embodiment, the salt of formula VII is selected from the group consisting of rosuvastatin (1-[(4-chlorophenyl)-phenyl-methyl]piperazinium salt and rosuvastatin (-)-1-[(4-chlorophenyl)-phenyl-methyl]piperazinium salt.

Salts of formula VII may be isolated in crystalline form.

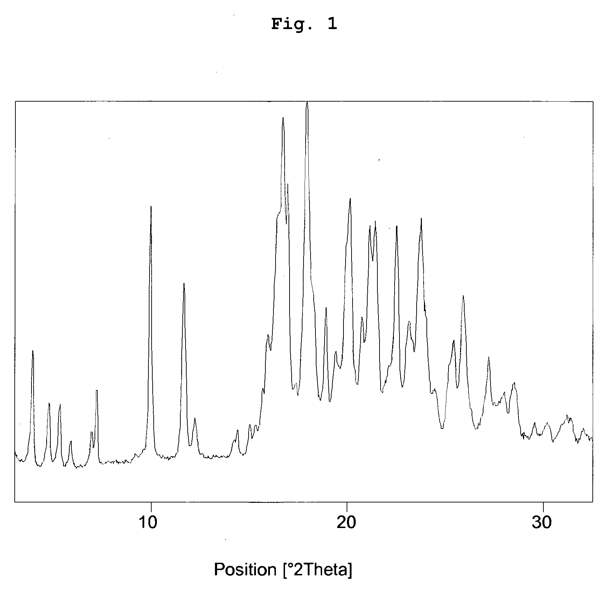

In one embodiment, the salt of formula VII is rosuvastatin 1-[(4-chlorophenyl)-phenyl-methyl]piperazinium salt which exhibits a powder x-ray diffraction pattern measured using CuKα-radiation having characteristic peak locations of 10.0, 16.7, 17.9 and 20.2 ± 0.2 degrees 2θ, particularly 10.0, 11.6, 16.7, 17.9, 20.2, 22.5 and 23.7 ± 0.2 degrees 2θ, more preferably exhibits a powder x-ray diffraction pattern substantially as shown in Fig. 1.

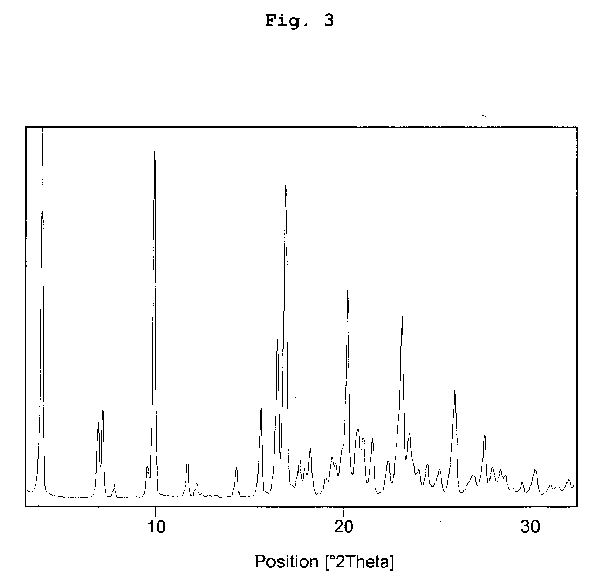

In another embodiment, the salt of formula VII is rosuvastatin (-)-1-[(4-chlorophenyl)-phenyl-methyl]piperazinium salt which exhibits a powder x-ray diffraction pattern measured using CuKα-radiation having characteristic peak locations of 3.9, 10.0 and 16.9 ± 0.2 degrees 2θ, particularly 3.9, 10.0, 16.5, 16.9, 20.2 and 23.2 ± 0.2 degrees 2θ, more preferably exhibits a powder x-ray diffraction pattern substantially as shown in Fig. 3.

The salt of formula VII can be prepared in a number of ways. In particular, the salt of formula VII can be prepared by reacting an amine of formula Z with a compound of formula I 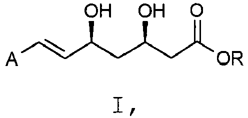 wherein A is as defined above and R is selected from the group consisting of H and (C₁-C₁₂)alkyl, preferably methyl and tert-butyl,
or a salt thereof.

In one embodiment, the amine of formula Z is reacted with a salt of the compound of formula I (i.e. R is a cation, preferably a metal ion) or with the free acid of the compound of formula I (i.e. R is H). For instance, a salt of a compound of formula I, such as the sodium salt obtained by NaOH catalyzed hydrolysis of the compound of formula II, can be converted to a salt of formula VII, such as the 1-[(4-chlorophenyl)-phenyl-methyl]piperazinium or (-)-1-[(4-chlorophenyl)-phenyl-methyl]piperazinium salt, by reaction with the corresponding amine or its solution in a suitable solvent. Alternatively, the free acid of a compound of formula I, such as rosuvastatin free acid, can be converted to such salt by reaction with the corresponding amine or its solution in a suitable solvent. The free acid, such as rosuvastatin free acid, can for instance be obtained by neutralization of the sodium salt with HCl and can be used either in isolated form or directly in form of a solution without intermediate isolation.

Salts of formula VII can be selectively crystallized or precipitated from organic solvents. Preferred solvents include alcohols such as methanol, ethanol, n-propanol, isopropanol, n-butanol, sec-butanol and isobutanol, ethers such as tert-butyl methyl ether and tetrahydrofuran (THF), esters such as ethyl acetate and isopropyl acetate, nitriles such as acetonitrile and mixtures thereof. Ethanol, tetrahydrofuran (THF), isopropyl acetate, tert-butyl methyl ether, acetonitrile and mixtures thereof are preferred. Ethanol, tetrahydrofuran (THF), isopropyl acetate and mixtures thereof are particularly preferred.

Furthermore, it has surprisingly been found that a compound of formula I wherein R is (C₁-C₁₂)alkyl, particularly methyl or tert-butyl, can be directly hydrolyzed by reaction with an amine of formula Z, particularly a piperazine, more particularly a piperazine of formula Z¹ such as 1-[(4-chlorophenyl)-phenyl-methyl]piperazine or (-)-1-[(4-chlorophenyl)-phenyl-methyl]piperazine, to obtain the respective organic ammonium salt. Hydrolysis may be achieved in a mixture of organic solvent and water, particularly in a ratio of 10:1 to 1:10, preferably 8:1 to 1:3, more preferably 5:1 to 1:1, most preferably 4:1 to 2:1. The organic solvent can be selected from acetonitrile, acetone, methanol, ethanol, isopropanol and mixtures thereof, wherein acetonitrile is preferred. The reaction may be carried out at a temperature of from 0 °C to the boiling point of the solvent mixture, particularly from 10 °C to 70 °C, more preferably 20 °C to 50 °C, most preferably 30 °C to 40 °C. The amine is typically used in an amount of 1 to 5 equivalents.

The salts of formula VII can be advantageously used in the preparation of pharmaceutically acceptable salts of HMG-CoA reductase inhibitors such as rosuvastatin, particularly amorphous and crystalline forms of rosuvastatin.

The invention also provides a process for the preparation of a pharmaceutically acceptable salt of rosuvastatin, which process comprises converting a rosuvastatin salt of formula VII to a pharmaceutically acceptable salt of rosuvastatin, particularly rosuvastatin calcium.

In one embodiment, the salt of formula VII, such as rosuvastatin 1-[(4-chlorophenyl)-phenyl-methyl]piperazinium salt or rosuvastatin (-)-1-[(4-chlorophenyl)-phenyl-methyl]piperazinium salt, is directly converted to the pharmaceutically acceptable salt, such as rosuvastatin calcium, for instance by addition of a calcium organic or inorganic salt, such as CaCl₂, Ca(OH)₂ or Ca acetate. In another embodiment, the salt of formula VII is first converted to a corresponding alkaline metal salt, particularly the sodium salt, which is subsequently converted to the pharmaceutically acceptable salt. In yet another embodiment, the salt of formula VII is converted to the corresponding free acid, such as rosuvastatin free acid, which may or may not be isolated in solid form, from which the pharmaceutically acceptable salt, such as rosuvastatin calcium, is formed directly or via an alkaline salt such as the sodium salt.

Preferably, the formation of the pharmaceutically acceptable salt, such as rosuvastatin calcium, for instance by reacting a solution of rosuvastatin sodium with a calcium salt, is carried out at a pH value of not less than 7. It has been found that at lower pH values of the reaction medium, rosuvastatin calcium is partially transformed to rosuvastatin lactone. Thus, in the final stage of the process of preparing the pharmaceutically acceptable salt the control of the pH value is important.

It is further preferred that formation of the pharmaceutically acceptable salt, such as rosuvastatin calcium, particularly in amorphous form, is carried out in the presence of at least one organic solvent. Preferably, the at least one organic solvent is used in an amount of 0.0001 to 2 vol%, particularly 0.01 to 1 vol%, more preferably from 0.1 to 0.7 vol% based on the total volume of the reaction mixture. The reaction is preferably carried out at a temperature of 10 to 40 °C, particularly 15 to 30 °C, more preferably 20 to 25 °C. Examples of suitable solvents include alcohols such as methanol, ethanol, isopropanol and isobutanol, ketones such as acetone and methyl ethyl ketone, esters such as ethyl acetate, n-propyl acetate, isopropyl acetate and isobutyl acetate, and ethers such as diethyl ether, diisopropyl ether, diisobutyl ether, methyl ethyl ether and tert-butyl methyl ether. Esters and ethers, particularly isopropyl acetate and tert-butyl methyl ether, are preferred. Isopropyl acetate is particularly preferred. It has surprisingly been found that carrying out the precipitation of a pharmaceutically acceptable salt of a compound of formula I, particularly amorphous rosuvastatin calcium, in the presence of a small amount of organic solvent allows preparation of such salt with a low specific surface area, particularly a specific surface area of below 40 m²/g, most preferably 0.5 m²/g to 40 m²/g. Due to the low specific surface area, such salt is less prone to degradation. Furthermore, low surface area is also indicative of low porosity. Low porosity has the advantage of reducing the number of pores in which impurities like NaCl can be caught which would be difficult to remove.

In particular, an aqueous solution of rosuvastatin sodium comprising a desired amount of organic solvent can be prepared by a process comprising extracting rosuvastatin free acid into an organic solvent, particularly an aromatic hydrocarbon such as toluene, an ether such as tert-butyl methyl ether, or an ester such as ethyl acetate or isopropyl acetate, more preferably toluene, from an acidified aqueous suspension of a salt of formula VII, such as rosuvastatin 1-[(4-chlorophenyl)-phenyl-methyl]piperazinium salt or rosuvastatin (-)-1-[(4-chlorophenyl)-phenyl-methyl]piperazinium salt, followed by formation and extraction of the formed rosuvastatin sodium salt from the organic solvent to a freshly added aqueous phase. Alternatively, the salt of formula VII, such as rosuvastatin 1-[(4-chlorophenyl)-phenyl-methyl]piperazinium salt or rosuvastatin (-)-1-[(4-chlorophenyl)-phenyl-methyl]piperazinium salt, can be directly converted to the corresponding sodium salt by combining the salt of formula VII with water, an organic solvent and an aqueous solution of sodium hydroxide. From the thus prepared solution of rosuvastatin sodium, solvents can be partially removed by distillation and/or pure water can be added until the desired amount of organic solvent, such as an ether or an ester, is achieved. Alternatively, any required amount of organic solvent may be added to the aqueous solution of the sodium salt.

The process may further comprise re-suspending the pharmaceutically acceptable salt of the compound of formula I, such as rosuvastatin calcium, in water to obtain a suspension containing 5 to 20 % by weight, preferably 10 % by weight, of said salt and macerating the suspension, preferably at a temperature of 5 to 25 °C, more preferably 10 to 15 °C. It has surprisingly been found that such process allows to efficiently remove any residual other salts, such as sodium salts without the need for wet milling.

In particular, the process may comprise
(a) suspending rosuvastatin calcium wet cake in water, to obtain a suspension containing 5 to 20 % by weight, preferably 10% by weight, of rosuvastatin calcium;
(b) stirring the suspension at a temperature of 5 to 25 °C, preferably 10 to 15 °C for 30 min to 2 hours, preferably 30 min to 1 hour, such as about 45 min; and
(c) recovering rosuvastatin calcium by filtration.

Maceration can be repeated until the desired result, such as a desired level of purity, is obtained. Thus the sodium content of for instance rosuvastatin calcium can be reduced from 4.0% by weight to 0.5 % by weight, preferably below 0.1 % by weight.

Rosuvastatin calcium may further be packaged in a package such as a polyethylene bag or hot sealed polyethylene laminated bag in nitrogen atmosphere, preferably in hot sealed polyethylene laminated bag in nitrogen atmosphere. Rosuvastatin calcium may be stored at temperatures ranging from 0 °C to room temperature, preferably 2 to 8 °C.

Instead of the amorphous rosuvastatin calcium, crystalline forms of rosuvastatin calcium may also be prepared. Such crystalline forms can be used in particular for synthesis and purification of other crystalline or amorphous forms of rosuvastatin calcium. Pharmaceutical compositions comprising the crystalline forms of rosuvastatin can also be prepared.

Also disclosed is a crystalline rosuvastatin calcium trihydrate having the formula

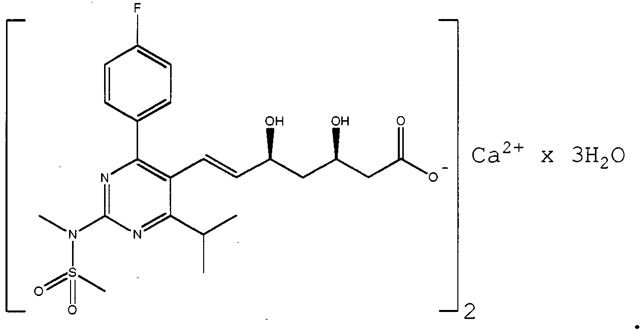

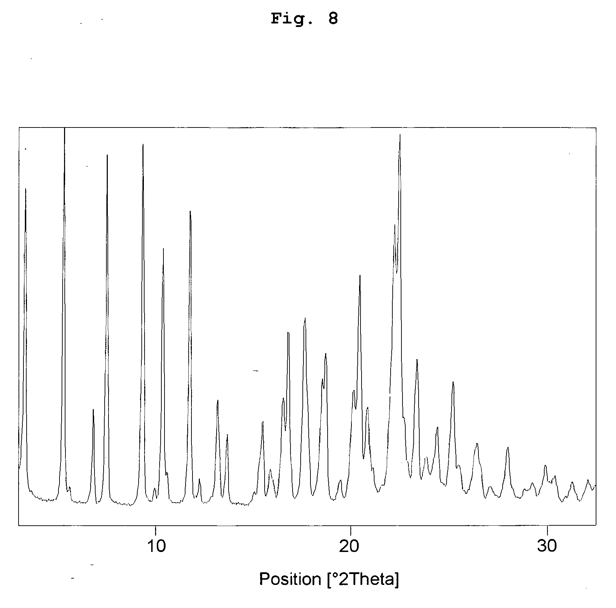

The crystalline rosuvastatin calcium trihydrate preferably exhibits a powder x-ray diffraction pattern measured using CuKα-radiation having characteristic peak locations of 5.3, 7.6, 9.4 and 23.4 ± 0.2 degrees 2θ, particularly 3.4, 5.3, 7.6, 9.4, 10.4, 11.8, 13.2, 17.7, 20.5 and 23.4 ± 0.2 degrees 2θ, more preferably exhibits a powder x-ray diffraction pattern substantially as shown in Fig. 8.

Typically, the crystalline rosuvastatin calcium trihydrate has a water content of 4.6 to 5.6 % by weight according to Karl Fischer analysis (European Pharmacopeia 5^{th} edition 2006, Method 2.5.12).

The crystalline rosuvastatin calcium trihydrate can be prepared by a process comprising crystallizing rosuvastatin calcium from a solution in water or a mixture of water with an organic solvent, particularly in a ratio of 0.05:1 to 1:0.05 (v:v), more preferably 0.1:1 to 1:0.1, most preferably 0.5:1 to 1:0.5. Preferably, the solvent is selected from isopropyl acetate, ethyl acetate and acetonitrile, more preferably from isopropyl acetate and ethyl acetate.

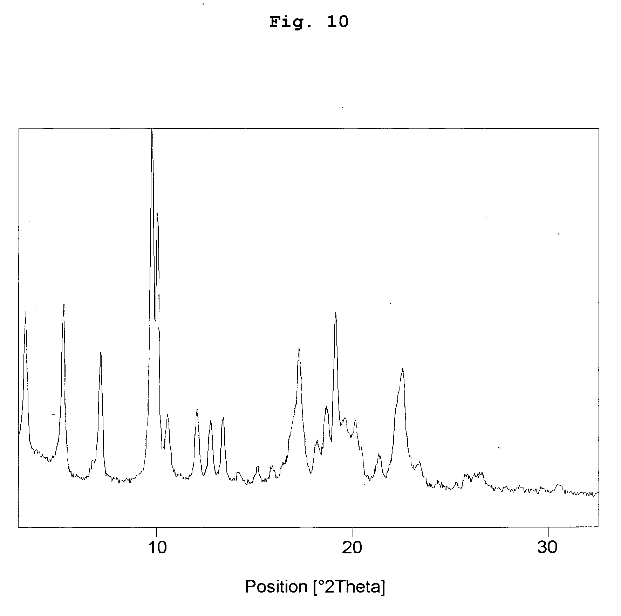

Also disclosed is a crystalline anhydrous rosuvastatin calcium. The crystalline anhydrous rosuvastatin calcium preferably exhibits a powder x-ray diffraction pattern measured using CuKα-radiation having characteristic peak locations of 3.4, 5.3, 9.7, 10.1 and 19.1 ± 0.2 degrees 2θ, particularly 3.4, 5.3, 7.1, 9.7, 10.1, 17.3, 19.1 and 22.6 ± 0.2 degrees 2θ, more preferably exhibits a powder x-ray diffraction pattern substantially as shown in Fig. 10.

The crystalline anhydrous rosuvastatin calcium can be prepared by a process comprising drying rosuvastatin calcium trihydrate in a vacuum dryer at a temperature of at least 50 °C, particularly at least 70 °C, most preferably at least 90 °C.

The invention will be further illustrated by way of the following examples.

### Examples

Measurements of chemical purity in the examples were performed by HPLC using the following conditions:
Column: Ascentis Express C8, 2.7 µm particles, 150 x 4.6 mm
Eluent A: 0.01 M ammonium acetate pH 3.8
Eluent B: acetonitrile : methanol = 70 : 30
Gradient:

| time (min) | % A | % B |
|---|---|---|
| 0 | 65 | 35 |
| 27 | 52 | 48 |
| 30 | 25 | 75 |
| 33 | 25 | 75 |
| 34 | 15 | 85 |
| 39 | 15 | 85 |
| 40 | 65 | 35 |

| | |
|---|---|
| Post time: | 4 min |
| Flow-rate: | 1.0 ml/min |
| Detection: | UV, 254 nm |
| Injection volume: | 5 µl |
| Column temperature: | 40 °C |
| Sample temperature: | 10 °C |
| Diluent: | 70% acetonitrile |

### Example 1-1

### a.) Tert-butyl 2-[(4R,6S)-6-(iodomethyl)-2,2-dimethyl-1,3-dioxan-4-yl]acetate (V)

Generally following the procedure described in US 5,093,363, reference example 1, tert-butyl 2-[(4R,6S)-6-(hydroxymethyl)-2,2-dimethyl-1,3-dioxan-4-yl]acetate (1 g, 3.84 mmol), imidazole (0.523 g, 7.68 mmol) and triphenylphosphine (2.01 g, 7.78 mmol) were added to toluene (11.5 mL) and cooled on ice. Under stirring, iodine (1.46 g, 5.80 mmol) was added portion-wise. After further stirring on ice for a few minutes, stirring was continued at room temperature for 2.5 h. The white precipitate formed was removed by filtration, and the filtrate was evaporated to dryness to give 1.295 g (91 %) of the title compound as a yellow oil. IR: 2978, 1727, 1380, 1368, 1261, 1203, 1158, 951, 844.

### b.) Tert-butyl2-{(4R,6S)-6-((triphenylphoshonium)methyl]-2,2-dimethyl-1,3-dioxan-4-yl}acetate iodide (IV)

*Tert-*butyl 2-[(4R,6S)-6-(iodomethyl)-2,2-dimethyl-1,3-dioxan-4-yl]acetate (1.61 g, 4.35 mmol), triphenylphosphine (1.15 g, 4.35 mmol) and acetonitrile (20 mL) were heated at reflux temperature under nitrogen atmosphere for 24 hours. The volatile components were evaporated to give 2.42 g (88 %) of the title compound as a viscous oil. IR: 3056, 2975, 2923, 1733, 1438, 1158, 1121, 724, 694.

### Example 1-2

### a.) Tert-butyl 2-{(4R,6S)-6-[(tert-butyldiphenylsilyloxy)methyl]-2,2-dimethyl-1,3-dioxan-4-yl}acetate

***Tert-*butyl** 2-[(4R,6S)-6-(hydroxymethyl)-2,2-dimethyl-1,3-dioxan-4-yl]acetate (20 mmol, 5.20g) was dissolved in dichloromethane (12 mL) at room temperature, imidazole (1.852 g) was added and the mixture was cooled on ice. Under stirring, tert-butyldiphenylchlorosilane (6.24 mL) was added. After stirred on ice for an additional 15 min, stirring was continued at room temperature for 20 min. The precipitate formed was removed by filtration, and the filtrate was successively washed with 5 % aqueous HCl, 5 % aqueous NaHCO₃ and water. The organic phase was dried with anhydrous Na₂SO₄, filtered and the solvent evaporated to obtain 9.689 g (97 %) of the title compound as an oily residue. IR: 2931, 2858, 1730, 1368, 1152, 1113, 702. ¹H-NMR (CDCl₃) : δ = 1.05 (s, 9H, tBu), 1.20 (dd, 1H, J = 24.31, 11.70 Hz), 1.35 (s, 3H), 1.43 (s, 3H), 1.45 (s, 9H), 1.69 (ddd, 1H, J = 12.75, 2.48 Hz), 2.31 (dd, 1H, J = 14.93, 6.04 Hz), 2.44 (dd, 1H, J = 14.93, 7.21 Hz), 3.54 (dd, 1H, J = 10.16, 6.05 Hz), 3.71 (dd, 1H, J = 10.15, 5.22 Hz), 3.99 (dddd, 1H, J = 6.04, 2.48 Hz), 4.22-4.31 (m, 1H), 7.34-7.42 (m, 6H), 7.66-7.73 (m, 4H).

### b.) Tert-butyl (3R,5S)-6-(tert-butyldiphenylsilyloxy)-3,5-dihydroxy-hexanoic acid

*Tert*-butyl 2-{(4R,6S)-6-[(tert-butyldiphenylsilyloxy)methyl]-2,2-dimethyl-1,3-dioxan-4-yl}acetate (9.439 g) was dissolved in 80 % aqueous acetic acid (50 mL) and stirred at room temperature for 4 h. The solvent was evaporated *in vacuo.* The oily residue was dissolved in ethyl acetate and washed with 5 % NaHCO₃ and water. The organic phase was dried with anhydrous Na₂SO₄, filtered and the solvent evaporated to give 9.08 g (99 %) of the title compound as an oily product. IR: 3428, 2931, 1727, 1428, 1152, 1113, 822, 741, 701, 608.

### c.) Tert-butyl 2-{(4R,6S)-6-[(tert-butyldiphenylsilyloxy)methyl]-2-phenyl-1,3-dioxan-4-yl}acetate

The oily residue obtained in the previous step was dissolved in dichloromethane (30 mL). Benzaldehyde dimethylacetal (3.3 mL) and pyridinium p-toluenesulfonate (0.5 g) were added, and the mixture was stirred at room temperature for 4 h. The solvent was removed *in vacuo* and the oily residue was purified by column chromatography on silica gel (ethyl acetate/hexane 1:4) to obtain 10.05 g (93 %) of the title compound. IR: 3070, 2931, 1729, 1428, 1368, 1154, 1113, 823, 742, 701. ¹H-NMR (CDCl₃) : δ = 1.06 (s, 9H), 1.46 (s, 9H), 1.81 (td, 1H, J = 12.94, 2.44 Hz), 2.31 (dd, 1H, J = 14.93, 6.03 Hz), 2.44 (dd, 1H, J = 15. 13, 7.48 Hz), 3.53 (dd, 1 H, J = 10.16, 6.09 Hz), 3.71 (dd, 1 H, J = 10.14, 5.20 Hz), 3.95-4.08 (m, 1H), 4,22-4.34 (m, 1H), 5.57 (s, 1H), 7.32-7.47 (m, 10H), 7.66-7.74 (m, 5H).

### d.) Tert-butyl 2-[(4R,6S)-6- (hydroxymethyl)-2-phenyl-1,3-dioxan-4-yl]acetate (VI)

The oily residue obtained in the previous step was dissolved in THF (20 mL). TBAF (4.7 g) was added and the mixture was stirred at room temperature for 4 h. The solution was poured into saturated aqueous sodium carbonate (40 mL), and the resulting mixture was extracted with dichloromethane (3 × 20 mL). The combined organic extracts were washed with water, dried with anhydrous Na₂SO₄, filtered and the solvent evaporated to give 5.42 g (98 %) of the title compound. IR: 3415, 2930, 2856, 1729, 1428, 1368, 1153, 1113, 876, 701.

### e.) Tert-butyl 2-[(4R,6S)-6-(iodomethyl)-2-phenyl-1,3-dioxan-4-yl]acetate (V)

The *tert*-butyl 2-[(4R,6S)-6-(hydroxymethyl)-2-phenyl-1,3-dioxan-4-yl]acetate (**VI**) obtained in the previous step was dissolved in toluene (20 mL). Imidazole (3.87 g) and triphenylphosphine (14.8 g) were added and the mixture was cooled on ice. Under stirring, iodine (10.8 g) was added portion-wise. After further stirring on ice for a few minutes, stirring was continued at room temperature for 2.5 h. The white precipitate formed was removed by filtration, and the solvent of the filtrate was evaporated in *vacuo* to give an oily product. The crude product was purified by dissolving the same in hexane, stirring the mixture at room temperature for 30 min and then filtered off the precipitate formed. The filtrate was evaporated to dryness to give 7.78 g (93 %) of the title compound as an oil. IR: 3069, 2929, 1728, 1434, 1113, 847, 743, 607, 541.

### f.) Tert-butyl 2-{(4R,6S)-6-[(triphenylphoshonium)methyl]-2-phenyl-1,3-dioxan-4-y1}acetate iodide (IV)

The tert-butyl 2-[(4R,6S)-6-(iodomethyl)-2-phenyl-1,3-dioxan-4-yl]acetate (**V**) obtained in the previous step was dissolved in acetonitrile (70 mL). Triphenylphosphine (4.04 g) was added, and the mixture was heated at reflux temperature for 17 h. The solvent was evaporated to give 10.97 g (81 %) of a white pasty product. IR: 3069, 1728, 1548, 1478, 1434, 1113, 1026, 880, 743, 607, 541.

### g.) Tert-butyl 2-[(4R,6S)-6-{(E)-2-[4-(4-fluorophenyl)-6-isopropyl-2-(N-methylmethylsulfon-amido)pyrimidin-5-yl)vinyl)-2-phenyl-1,3-dioxan-4-yl]acetate (II)

*Tert*-butyl 2-{(4*R*,6*S*)-6-[(triphenylphosphonium)methyl]-2-phenyl-1,3-dioxan-4-yl}acetate iodide (**IV**) (10.95 g) was dissolved in THF (30 mL) and cooled on ice Potassium tert-butoxide (5.85 g) was added and the mixture was stirred for 30 min. A solution of N-[4-(4-fluorophenyl)-5-formyl-6-isopropylpyrimidin-2-yl]-N-methylmethanesulfonamide (**III)** (5.65 g) in THF (20 mL) was added dropwise under stirring. The mixture was allowed to warm to room temperature and was further stirred at this temperature for 12 h. The mixture was quenched with saturated aqueous NH₄Cl and diluted with Et₂O/H₂O. The layers were separated, and the aqueous layer was extracted (3 × Et₂O). The combined organic phases were dried with anhydrous Na₂SO₄, filtered, and evaporated to give an oily product, which was purified by column chromatography on silica gel (ethyl acetate/hexane 1:4) to obtain 9.56 g (75 %) of the title compound.

### h.) Tert-butyl (3R,5S,E)-7-[4-(4-fluorophenyl)-6-isopropyl-2-(N-methylmethylsulfonamido)-pyrimidin-5-yl]-3,5-dihydroxyhept-6-enoate (I)

*Tert*-butyl 2-[(4*R*,6*S*)-6-{(*E*)-2-[4-(4-fluorophenyl)-6-isopropyl-2-(*N*-methylmethylsulfon-amido)pyrimidin-5-yl}vinyl)-2-phenyl-1,3-dioxan-4-yl]acetate (**II**) (9.55 g) was dissolved in 80% acetic acid (50 mL) and stirred 3 h at elevated temperature (40 - 60 °C). The solvent was evaporated. The oily residue was dissolved in ethyl acetate (40 mL) and washed with 5 % aqueous NaHCO₃ and water. The organic phase was dried with anhydrous Na₂SO₄, filtered and the solvent evaporated to give 7.79 g (95 %) of the title compound as an oily product.

Under similar conditions also *methyl* (3*R*,5*S*,*E*)-7-[4-(4-fluorophenyl)-6-isopropyl-2-(N-methylmethylsulfonamido)-pyrimidin-5-yl]-3,5-dihydroxyhept-6-enoate was prepared.

### Example 1-3

### (E)-7-{4-(4-fluorophenyl)-6-isopropyl-2-[methyl(methylsulfonyl)amino]-pyrimidin-5-yl}-(3R,5S)-3,5-dihydroxy-hept-6-enoic acid sodium salt (Rosuvastatin-Na, IA-Na)

Generally following the procedure disclosed in EP 0 521 471, rosuvastatin (200 mmol) was dissolved in ethanol (1347 mL). Under stirring and cooling, 0.1 M NaOH (1672 mL) was slowly added. After complete addition of the NaOH, stirring was continued at room temperature for 1 h. The solvent was evaporated under reduced pressure, and the oily residue was treated with ether (400 mL). The precipitated crystals of the sodium salt of rosuvastatin were filtered off. Optionally, if crystals were not formed, the ether was evaporated and crystals gradually formed from the obtained foamed oil. IR: 3421, 2968, 1605, 1546, 1510, 1381, 1155, 963, 834, 775, 519.

### Example 1-4

### Rosuvastatin free acid solution

Rosuvastatin methyl ester (100 g) was dissolved in ethanol (2000 mL), and while stirring at 0 °C sodium hydroxide aqueous solution (0.1 M, 2000 mL) was added dropwise. After stirring at 0 °C for 30 min, stirring was continued at room temperature for 1 h. The mixture was concentrated to ∼ 1000 mL. Isopropyl acetate (2000 mL) was added, and hydrochloric acid (1 M, 200 mL) was added under vigorous stirring. After stirring for 1 h, the phases were separated and the organic phase was dried with azeotropic distillation.

### Example 1-5

### Rosuvastatin free acid

Rosuvastatin methyl ester (1 g) was dissolved in ethanol (20 mL), and sodium hydroxide (0.1 M, 20 mL) was added dropwise under stirring on ice. After stirring on ice for 30 min, stirring was continued at room temperature for 1 h. The reaction mixture was concentrated to ∼ 10 mL. Isopropyl acetate was (20 mL) added, and hydrochloric acid (1M, 2 mL) was added under vigorous stirring on ice. After stirring for 1h, the phases were separated and the organic phase was dried with anhydrous Na₂SO₄ and filtered. Removal of volatile components furnished rosuvastatin free acid as an oily substance, which gradually crystallised.

### Example 2-1

### Rosuvastatin 1-[(4-chlorophenyl)-phenyl-methyl]piperazinium salt (Rosuvastatin CPPMP salt)

### A.) Preparation of seeding crystals

a.) To a solution of rosuvastatin free acid (0.96 g) in isopropyl acetate (20 mL), a solution of 1-[(4-chlorophenyl)-phenyl-methyl]piperazine (0.58 g) in isopropyl acetate (5 mL) was added at a temperature between 50 °C and 60 °C. After the addition, stirring was continued at the same temperature for 30 minutes. The mixture was then slowly cooled to room temperature (between 20 °C and 25 °C) and stirring was continued for another 4 h. Rosuvastatin 1-[(4-chlorophenyl)-phenyl-methyl]piperazinium salt precipitated from the reaction mixture as a white crystalline solid, which was filtered off and dried at 30 °C *in vacuo.* Yield: 93 %. The crystals of rosuvastatin 1-[(4-chlorophenyl)-phenyl-methyl]piperazinium salt were re-crystallized from a mixture of THF and ethanol.
b.) To a solution of Rosuvastatin free acid (0.96 g) in isopropyl acetate (20 mL), a solution of 1-[(4-chlorophenyl)-phenyl-methyl]piperazine (0.58 g) in isopropyl acetate (5 mL) was added at room temperature. After the addition, stirring was continued for 6 h. Rosuvastatin 1-[(4-chlorophenyl)-phenyl-methyl]piperazinium salt precipitated from the reaction mixture as a white crystalline solid, which was filtered off and dried at 30 °C *in vacuo.* Yield: 93%. The crystals of rosuvastatin 1-[(4-chlorophenyl)-phenyl-methyl]piperazinium salt were re-crystallized from a mixture of THF and ethanol.

### B.) Crystallization of rosuvastatin 1-[(4-chlorophenyl)-phenyl-methyl]piperazinium salt with seeding

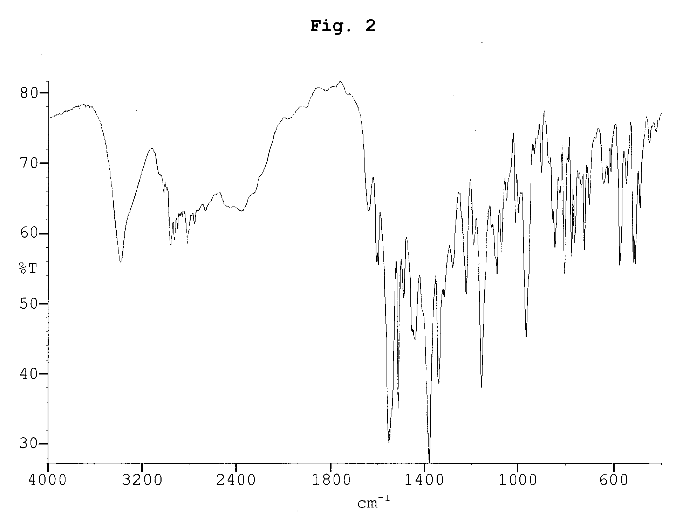

1-[(4-chlorophenyl)-phenyl-methyl]piperazine (3.7 g) was dissolved in isopropyl acetate (40 mL). 2 vol% of this solution was added to a solution of rosuvastatin free acid (17 g) in isopropyl acetate (133 mL) at room temperature. The obtained mixture was seeded with 0.5 % of pure rosuvastatin 1-[(4-chlorophenyl)-phenyl-methyl]piperazinium salt. The rest of the prepared solution of 1-[(4-chlorophenyl)-phenyl-methyl]piperazine in isopropyl acetate was slowly added over approx. 5 h. After the addition, stirring was continued for another hour. Rosuvastatin 1-[(4-chlorophenyl)-phenyl-methyl]piperazinium salt precipitated from the reaction mixture as a white, crystalline solid, which was filtered off, washed with isopropyl acetate (25 mL) and dried at 40 °C *in vacuo.* Yield: 90 %. The XRPD pattern, which was recorded on a Phillips PW3040/60 X'Pert PRO diffractometer using CuKα radiation (1,541874 Å), is shown in Fig. 1. The FT-IR spectrum, which was recorded on a FT-IR System SPECTRUM 1000 Perkin-Elmer [4000-400 cm-1, Resolution 4 cm-1, KBr tbl.], is shown in Fig. 2.

### C.) Conversion of rosuvastatin 1-[(4-chlorophenyl)-phenyl-methyl]piperazinium salt to rosuvastatin sodium and calcium salts

Rosuvastatin 1-[(4-chlorophenyl)-phenyl-methyl]piperazinium salt (1.537 g, 2 mmol) was dissolved in a mixture of water (10 mL) and isopropyl acetate or dichloromethane (20 mL). Sodium hydroxide solution (1 M) was added under vigorous stirring until pH 9 was reached. The phases were separated, and the aqueous phase containing rosuvastatin sodium salt was further used for the preparation of rosuvastatin calcium according to the procedures described below.

### Example 2-2

### Rosuvastatin (-)-1-[(4-chlorophenyl)-phenyl-methyl]piperazinium salt (Rosuvastatin (-) -CPPMP salt)

### A.) Preparation of seeding crystals

a.) To a solution of rosuvastatin free acid (0.96 g) in isopropyl acetate (20 mL), a solution of (-)-1-[(4-chlorophenyl)-phenyl-methyl]piperazine (0.58 g) in isopropyl acetate (5 mL) was added at a temperature between 50 °C and 60 °C. After the addition, stirring was continued at the same temperature for 30 minutes. The mixture was then slowly cooled to room temperature (between 20 °C and 25 °C) and stirring was continued for another 4 h. Rosuvastatin (-)-1-[(4-chlorophenyl)-phenyl-methyl]piperazinium salt precipitated from the reaction mixture as a white crystalline solid, which was filtered off and dried at 30 °C *in vacuo.* Yield: 93 %. The crystals of rosuvastatin (-)-1-[(4-chlorophenyl)-phenyl-methyl]piperazinium salt were re-crystallized from a mixture of THF and ethanol.
b.) To a solution of Rosuvastatin free acid (0.96 g) in isopropyl acetate (20 mL), a solution of (-)-1-[(4-chlorophenyl)-phenyl-methyl]piperazine (0.58 g) in isopropyl acetate (5 mL) was added at room temperature. After the addition, stirring was continued for 6 h. Rosuvastatin (-)-1-[(4-chlorophenyl)-phenyl-methyl]piperazinium salt precipitated from the reaction mixture as a white crystalline solid, which was filtered off and dried at 30°C *in vacuo.* Yield: 93%. The crystals of rosuvastatin (-)-1-[(4-chlorophenyl)-phenyl-methyl]piperazinium salt were re-crystallized from a mixture of THF and ethanol.

### B.) Crystallization of Rosuvastatin (-)-1-[(4-chlorophenyl)-phenyl-methyl]piperazinium salt with seeding

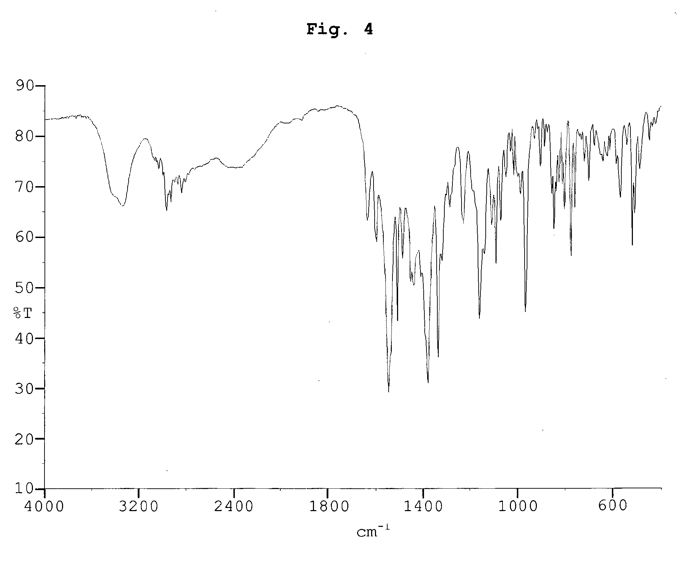

a.) (-)-1-[(4-chlorophenyl)-phenyl-methyl]piperazine (3.7 g) was dissolved in isopropyl acetate (40 mL). 2 vol% of this solution was added to a solution of rosuvastatin free acid (17 g) in isopropyl acetate (133 mL) at room temperature. The obtained mixture was seeded with 0.5 % of pure rosuvastatin (-)-1-[(4-chlorophenyl)-phenyl-methyl]piperazinium salt. The rest of the prepared solution of (-)-1-[(4-chlorophenyl)-phenyl-methyl]piperazine in isopropyl acetate was slowly added over approx. 5 h. After the addition, stirring was continued for another hour. Rosuvastatin (-)-1-[(4-chlorophenyl)-phenyl-methyl]piperazinium salt precipitated from the reaction mixture as a white, crystalline solid, which was filtered off, washed with isopropyl acetate (25 mL) and dried at 40 °C *in vacuo.* Yield: 90 %. The XRPD pattern, which was recorded on a Phillips PW3040/60 X'Pert PRO diffractometer using CuKα radiation (1,541874 Å), is shown in Fig. 3. The FT-IR spectrum, which was recorded on a FT-IR System SPECTRUM 1000 Perkin-Elmer [4000-400 cm-1, Resolution 4 cm-1, KBr tbl.], is shown in Fig. 4.
The purity of the rosuvastatin (-)-1-[(4-chlorophenyl)-phenyl-methyl]piperazinium salt obtained as above is depicted in Table 1.

**Table 1: Purity of rosuvastatin (-)-CPPMP salt**

| | HPLC purity | |
|---|---|---|
| | Starting material | After purification |
| **Impurity A** | 0.1228 | 0.0237 |
| **Impurity B** | 0.3003 | 0.041 |
| **Impurity C** | 2.1717 | 0.023 |
| **Rosuvastatin (-)-CPPMP salt** | 97.089 | 99.9 |

| | | |
|---|---|---|
| Impurity A: Rosuvastatin (3R, 5R)-diastereoisomer Impurity B: methyl 7-[4-(4-fluorophenyl)-6-isopropyl-2-(N-methyl-N-methylsulfonylamino)pyrimidin-5-yl]-(3R)-3-hydroxy-5-oxo-(E)-6-heptenate Impurity C: Rosuvastatin lactone | | |

b.) Rosuvastatin (-)-1-[(4-chlorophenyl)-phenyl-methyl]piperazinium salt (30 g) was suspended in ethanol (330 mL). The mixture was heated until reflux was achieved. The solution was hot filtered and cooled to about 50 °C and seeded with pure rosuvastatin (-)-1-[(4-chlorophenyl)-phenyl-methyl]piperazinium salt (0.3 g). The obtained suspension was slowly cooled to below 10 °C and filtered off. The filter cake was washed with ethanol (10 mL) and dried *in vacuo* at 40 °C to obtain rosuvastatin (-)-1-[(4-chlorophenyl)-phenyl-methyl]piperazinium salt.

### C.) Direct preparation of rosuvastatin (-)-1-[(4-chlorophenyl)-phenyl-methyl]piperazinium salt from rosuvastatin methyl ester

Rosuvastatin methyl ester (10 g) was dissolved in a mixture of acetonitrile (150 mL) and water (50 mL). (-)-1-[(4-chlorophenyl)-phenyl-methyl]piperazine (12 g) was added while stirring at 0 to 10 °C. The temperature was raised to 40 °C and the mixture was stirred overnight. The mixture was concentrated to half its previous volume by evaporation under reduced pressure. The remaining mixture was cooled to 10 °C and seeded with rosuvastatin (-)-1-[(4-chlorophenyl)-phenyl-methyl]piperazinium salt. The obtained solid was filtered off and dried at 40 °C *in vacuo* to obtain (-)-1-[(4-chlorophenyl)-phenyl-methyl]piperazinium salt.

### D.) Conversion of rosuvastatin (-)-1-[(4-chlorophenyl)-phenyl-methyl]piperazinium salt into rosuvastatin sodium and calcium salts

Rosuvastatin (-)-1-[(4-chlorophenyl)-phenyl-methyl]piperazinium salt (1.537 g, 2 mmol) was dissolved in a mixture of water (10 mL) and isopropyl acetate or dichloromethane (20 mL). Sodium hydroxide solution (1 M) was added under vigorous stirring until pH 9 was reached. The phases were separated, and the aqueous phase containing rosuvastatin sodium salt was further used for the preparation of rosuvastatin calcium according to the procedures described below.

### Example 3-1

### Rosuvastatin calcium - General method

a.) Rosuvastatin ammonium salt (2 mmol) was suspended in water (10 mL). Sodium hydroxide aqueous solution (1 M, 1 eqiv.) and isopropyl acetate (10 mL) were added and the mixture was stirred at room temperature for 1 h. The phases were separated and the aqueous phase was partially evaporated to remove isopropyl acetate traces.
b.) Rosuvastatin ammonium salt (2 mmol) was dissolved in a mixture of water (10 mL) and isopropyl acetate or dichloromethane (20 mL). Sodium hydroxide solution (1 M) was added under vigorous stirring until pH 9 was reached. The phases were separated.

To the aqueous solution of rosuvastatin sodium salt obtained either by the procedure under a.) or b.), calcium chloride (1 M in water) was added dropwise, and the mixture was stirred at room temperature for 2 h. Product precipitated as a white solid, which was filtered off, washed with cold water and dried 2 h in *vacuo* at 50°C.

### Example 3-3

### Rosuvastatin calcium I (R=Ca_{1/2})

Rosuvastatin ammonium salt (2 mmol) was suspended in water (10mL). Calcium chloride (1 M in water) was added dropwise, and the mixture was stirred at room temperature for 2 h. Product precipitated as a white solid, which was filtered off, washed with cold water and dried for 2 h *in vacuo* at 50 °C.

The same process was applied to rosuvastatin 1-[(4-chlorophenyl)-phenyl-methyl]piperazinium salt and rosuvastatin (-)-1-[(4-chlorophenyl)-phenyl-methyl]piperazinium salt.

### Example 3-4

### (E)-7-{4-(4-fluorophenyl)-6-isopropyl-2-[methyl(methylsulfonyl) amino]-pyrimidin-5-yl}-(3R,5S)-3,5-dihydroxy-hept-6-enoic acid calcium salt (Rosuvastatin-Ca, IA-Ca)

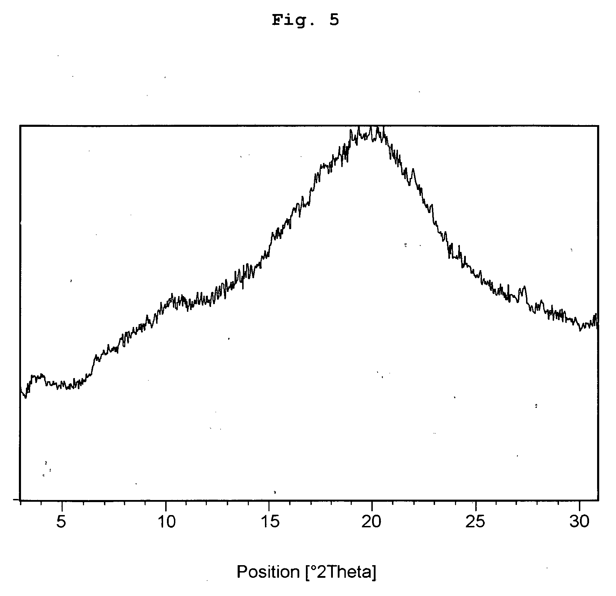

Rosuvastatin-Na (101 g, 200 mmol) was dissolved in water (1011 mL) at room temperature under a nitrogen atmosphere. 1 M CaCl₂ (202 mL) was added slowly (1 mL/min), and the mixture was stirred at room temperature for 2 h. Precipitated product was filtered off, washed with water and dried to obtain 100 g of powdered amorphous calcium salt of rosuvastatin. IR: 3400, 2967, 1604, 1548, 1510, 1438, 1381, 1155, 965, 844, 776. XRPD analysis proved that the obtained title compound was amorphous. The corresponding XRPD pattern, which was recorded on a Phillips PW3040/60 X'Pert PRO diffractometer using CuKα radiation (1,541874 Å), is shown in Fig. 5.

### Example 3-5

### (E)-7-{4-(4-fluorophenyl)-6-isopropyl-2-(methyl(methylsulfonyl)amino]-pyrimidin-5-yl)-(3R,5S)-3,5-dihydroxy-hept-6-enoic acid calcium salt (Rosuvastatin-Ca, IA-Ca)

A reactor was purged with nitrogen. Amorphous or crystalline rosuvastatin calcium salt (80 g) was suspended in isopropyl acetate (1600 mL) and water (800 mL), and the mixture was cooled to 5 °C. Hydrochloric acid (1 M, 180 mL) was added over 1.h until the pH of the mixture reached a value of 2. The mixture was stirred for 1 h at 5 °C. The organic phase was separated and water (800 mL) was added to the organic phase. A solution of sodium hydroxide (1 M, 165 mL) was added until the pH of the aqueous phase reached a value of 10. The aqueous phase containing rosuvastatin sodium was concentrated under reduced pressure at 40 °C to a volume of 800 mL. The solution was cooled to 25 °C and calcium chloride solution (1 M, 100 mL) was added over 2 hours under stirring. The suspension was stirred at 25 °C for 2 h. Precipitated product was filtered off, washed with water and dried in a fluid-bed drier at 30°C for 30 minutes, followed by 30 minutes at 35 °C and finally 30 minutes at 40 °C. Yield: 88%. The moisture content in the final product as determined by Karl-Fischer analysis was below 2 %. XRPD analysis proved that the obtained compound was amorphous. The specific surface area as measured on TriStar 3000 instrument using 6 point BET analysis with nitrogen as the adsorbent gas was 2.5 m²/g.

### Example 3-6

### Preparation of Rosuvastatin calcium from rosuvastatin methyl ester

a.) Rosuvastatin methyl ester (25 g, purity 98 %) was dissolved in ethanol (500 mL), and sodium hydroxide aqueous solution (0.1 M, 500 mL) was slowly added under stirring at about 0 °C. After 30 min of stirring at 0 °C, stirring was continued at room temperature. After 1 h of stirring, the mixture was concentrated to about 250 mL. The obtained solution was cooled to about 5 °C, and isopropyl acetate (500 mL) was added. Hydrochloric acid (1 M, 50 mL) was added under vigorous stirring and the mixture was stirred for 1 h. The phases were separated and the organic phase was dried with azeotropic distillation.
b.) (-)-1-[(4-chlorophenyl)-phenyl-methyl]piperazine (14.5 g) was dissolved in isopropyl acetate (40 mL). 2 vol% of this solution was added to the solution of rosuvastatin free acid in isopropyl acetate obtained in step a.). The obtained mixture was seeded at room temperature with 0.5 % of pure (-)-1-[(4-chlorophenyl)-phenyl-methyl]piperazinium salt. The rest of the prepared solution of (-)-1-[(4-chlorophenyl)-phenyl-methyl]piperazine in isopropyl acetate was slowly added over approx. 5 h. After the addition, stirring was continued for another hour. Rosuvastatin (-)-1-[(4-chlorophenyl)-phenyl-methyl]piperazinium salt precipitated from the reaction mixture as a white crystalline solid, which was filtered off, washed with isopropyl acetate (20 mL) and dried at 40 °C *in vacuo.*
c.) The obtained rosuvastatin (-)-1-[(4-chlorophenyl)-phenyl-methyl]piperazinium salt (7.65 g) was suspended in a mixture of water (77 mL) and toluene (77 mL). Sodium hydroxide solution (1 M, 10 mL) was added under vigorous stirring at room temperature to dissolve the solid. The phases were separated and the aqueous phase containing rosuvastatin sodium salt was further extracted with toluene (38 mL). The aqueous phase was concentrated under reduced pressure to about 50 mL.
d.) Isopropyl acetate (0.2 mL, 0.4 vol%) was added to the solution. 1 M calcium chloride (6.1 mL) was added at 25 °C over approximately 2 h. The obtained suspension was stirred for another hour. The solid was filtered off and washed with water (10 mL). The wet product was suspended in water (45 mL) at about 5 °C and stirred for 30 min. The solid was filtered off and washed with water (10 mL). The product was dried in a fluid-bed drier until the content of water was less than 20 % and then in a vacuum drier. XRPD analysis proved that the obtained compound was amorphous.

Rosuvastatin calcium was packed in a hot sealed polyethylene laminated bag under nitrogen atmosphere and stored at 2 to 8°C.

### Example 3-7

### Influence of pH on formation of rosuvastatin lactone

At lower pH values rosuvastatin calcium can be partially transformed into rosuvastatin lactone. Formation of lactone impurity in rosuvastatin calcium depending on the pH of the rosuvastatin sodium solution used for preparation of the calcium salt was studied. 1 M calcium chloride was added to aqueous solutions of rosuvastatin sodium at about 25 °C at various pH values. The results are as follows:

| pH | Rosuvastatin lactone [%] |
|---|---|
| 9 | 0.01 |
| 8 | 0.03 |
| 7 | 0.05 |
| 6 | 0.17 |

### Example 3-8

### Preparation and maceration of rosuvastatin calcium with water

140 mL of rosuvastatin sodium aqueous solution (80 mg/mL) was charged in a 200 mL reactor with overhead stirrer, and 0.6 mL of isopropyl acetate was added. Precipitation was performed at 25 °C with ∼ 156 g of 1 M calcium chloride solution which was added over 2 hours. The suspension was further stirred at the same temperature for 2 h and filtered. 17.81 g of rosuvastatin calcium wet cake was obtained.

Half of the obtained rosuvastatin calcium was dried *in vacuo* for 10 h at 30 °C and 5 h at 40 °C. 4.72 g of rosuvastatin calcium with a NaCl content of 0.96 % by weight was obtained.

The other half of the obtained rosuvastatin calcium was resuspended in 45 mL of water at 5 °C and stirred for 45 min. The product was recovered and dried *in vacuo* for 10 h at 30 °C and 5 h at 40 °C. 4.2 g of dry product with a NaCl content of 0.18% by weight was obtained.

### Example 4

### Preparation of low surface area amorphous rosuvastatin calcium by the addition of organic solvents

An aqueous solution of rosuvastatin sodium was prepared according to the example 3-6, step c.). After complete removal of organic solvents from the aqueous solution by evaporation, 600 mL of the solution were taken from the reactor. Three experiments A, B and C (140 mL each) were performed with varying amounts of isopropyl acetate added to the solution during the precipitation step as shown in Table 3.

In each experiment, precipitation was performed in a 200 mL reactor at 25 °C. 14.7 g of 1 M calcium chloride solution was added over 2 h. The suspension was further stirred at that temperature for 2 h and filtered. Product was dried in vacuum for 10 h at 30 °C and for 5 h at 40 °C. Specific surface areas of obtained the product were measured with a gas sorption system based on nitrogen adsorption, using the 6-point Brunauer, Emmett and Teller (BET) method. The obtained specific surface area values show a correlation of specific surface and filterability of product with the amount of isopropyl acetate.

**Table 3: Specific surface area rosuvastatin calcium salt**

| **Experiment** | A No addition of iPrOAc | B 0.4 vol% of iPrOAc | C 0.7 vol% of iPrOAc |
|---|---|---|---|
| **Specific surface area (m²/g)** | 40.0 | 3.6 | 0.5 |

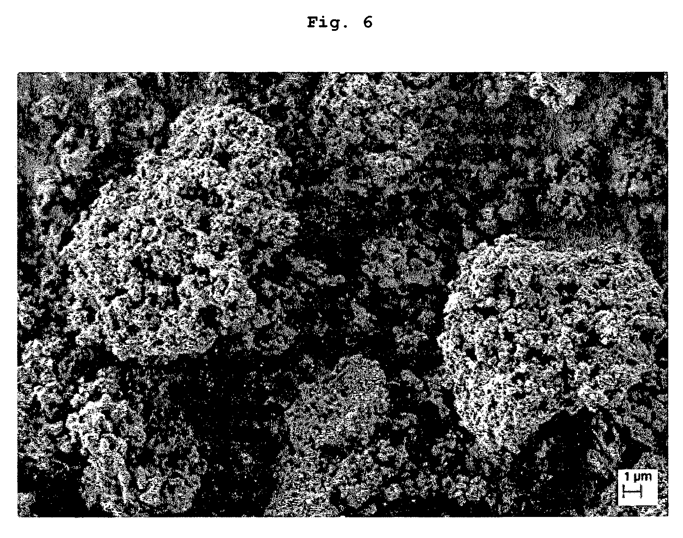

Fig. 6 represents a microphotograph of the amorphous rosuvastatin calcium obtained according to Experiment A. This microphotograph was recorded on a Scanning Electron microscope Zeiss Supra 35VP using 10.000x magnification.

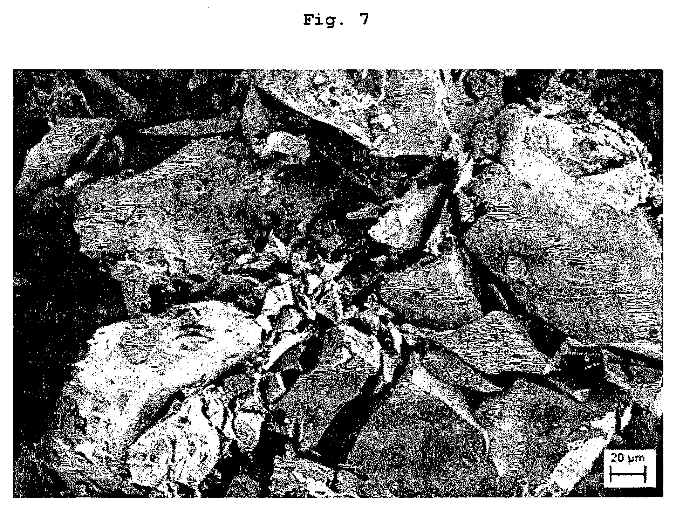

Fig. 7 represents a microphotograph of amorphous Rosuvastatin calcium obtained according to Experiment B. This microphotograph was recorded on a Scanning Electron microscope Zeiss Supra 35VP using 1000x magnification.

### Example 5

### Crystalline rosuvastatin calcium trihydrate

### A.) Preparation of seeding crystals

a.) Rosuvastatin calcium (5 g) was suspended in a mixture of ethyl acetate (55 mL) and water (55 mL). The suspension was heated until the solid was dissolved. The mixture was then slowly cooled to below 10 °C, during which cooling a solid was formed. The solid was filtered off and dried at 40 °C to obtain rosuvastatin calcium trihydrate.
b.) Rosuvastatin calcium (5 g) was suspended in a mixture of isopropyl acetate (55 mL) and water (55 mL). The suspension was heated until the solid was dissolved. The mixture was then slowly cooled to below 10 °C, during which cooling a solid was formed. The solid was filtered off and dried at 40 °C to obtain rosuvastatin calcium trihydrate.
c.) Rosuvastatin calcium (1 g) was suspended in a mixture of isopropyl acetate (20 mL) and water (10 mL). The solvent was slowly evaporated, and a solid crystallized from the residue. The solid was filtered off and dried at 40 °C to obtain rosuvastatin calcium trihydrate.

### B.) Preparation of Rosuvastatin calcium trihydrate with seeding

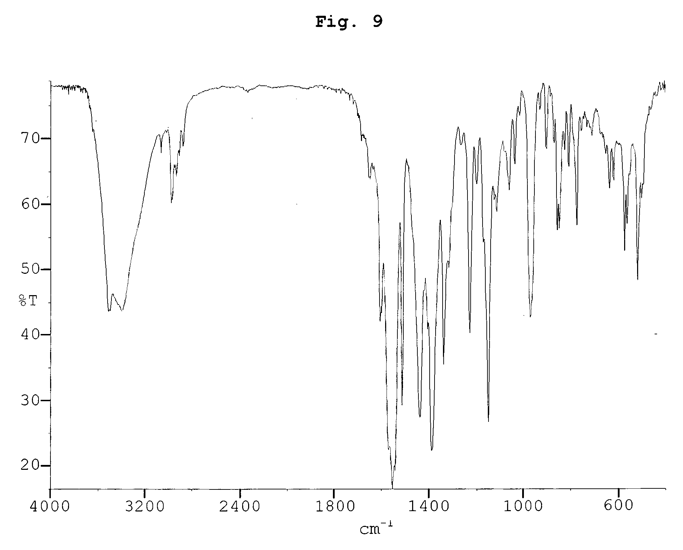

a.) Rosuvastatin calcium (20 g) was suspended in a mixture of isopropyl acetate (220 mL) and water (220 mL). The suspension was heated until the solid was dissolved. The mixture was then seeded with rosuvastatin calcium trihydrate. The obtained suspension was slowly cooled to below 10 °C. The solid was filtered off and dried at 40 °C to obtain rosuvastatin calcium trihydrate.
b.) Rosuvastatin calcium (20 g) was suspended in a mixture of ethyl acetate (220 mL) and water (220 mL). The suspension was heated until solid was dissolved. The mixture was then seeded with rosuvastatin calcium trihydrate. The obtained suspension was slowly cooled to below 10 °C. The solid was filtered off and dried at 40 °C to obtain rosuvastatin calcium trihydrate. The XRPD pattern, which was recorded on a Phillips PW3040/60 X'Pert PRO diffractometer using CuKα radiation (1,541874 Å), is shown in Fig. 8. The FT-IR spectrum, which was recorded on a FT-IR System SPECTRUM 1000 Perkin-Elmer [4000-400 cm-1, Resolution 4 cm-1, KBr tbl.], is shown in Fig. 9.
c.) Rosuvastatin calcium (20 g) was suspended in a mixture of acetonitrile (220 mL) and water (220 mL). The suspension was heated until the solid was dissolved. The mixture was then seeded with rosuvastatin calcium trihydrate. The obtained suspension was slowly cooled to below 10 °C. The solid was filtered off and dried at 40 °C to obtain rosuvastatin calcium trihydrate.
d.) Rosuvastatin calcium (2 g) was suspended in water (30 mL) at room temperature. The suspension was stirred for 24 h and cooled to below 10 °C. The solid was filtered off to obtain rosuvastatin calcium trihydrate.

### C.) Direct conversion of rosuvastatin (-)-1-[(4-chlorophenyl)-phenyl-methyl]piperazinium salt into rosuvastatin calcium trihydrate

Rosuvastatin (-)-1-[(4-chlorophenyl)-phenyl-methyl]piperazinium salt (5 g) was suspended in a mixture of acetonitrile (25 mL) and water (25 mL). The suspension was heated until a solution was obtained. Calcium chloride (1 M, 3.6 mL) was slowly added. The mixture was seeded with rosuvastatin calcium trihydrate (50 mg). The obtained suspension was slowly cooled to below 20 °C. The solid was filtered off and dried at 40 °C to obtain rosuvastatin calcium trihydrate.

### Example 6

### Crystalline anhydrous rosuvastatin calcium

Crystalline rrosuvastatin calcium trihydrate as obtained in Example 5 was dried in a vacuum dryer a temperature of 90 °C for 30 minutes to obtain crystalline anhydrous rrosuvastatin calcium. The XRPD pattern, which was recorded on a Phillips PW3040/60 X'Pert PRO diffractometer using CuKα radiation (1,541874 Å), is shown in Fig. 10.

## Claims

1. A salt of formula VII 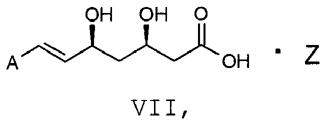 wherein
A is 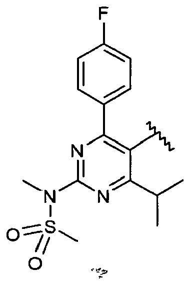 and
Z is a 4-(C₁-C₁₂-alkyl)piperazine wherein the C₁-C₁₂-alkyl group comprises at least one further substituent independently selected from the group consisting of unsubstituted or substituted aryl, heteroaryl, (C₃-C₆)cycloalkyl and (C₃-C₆)heterocycloalkyl.

2. Salt according to claim 1, wherein Z is an amine of formula Z¹ 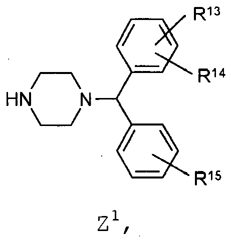 wherein R¹³, R¹⁴ and R¹⁵ are independently selected from H, halogen, (C₁-C₆)alkyl, (C₁-C₆)alkoxy, CH₂F, CHF₂ and CF₃, wherein halogen is independently selected from F, Cl, Br and I.

3. Salt according to claim 2, wherein
R¹³ is selected from 4-halogen, 4-(C₁-C₆)alkyl, 4-(C₁-C₆)alkoxy, 4-CH₂F, 4-CHF₂ and 4-CF₃;
R¹⁴ is selected from H, 3- or 5-halogen, 3- or 5-(C₁-C₆)alkyl, 3- or 5-(C₁-C₆)alkoxy, 3- or 5-CH₂F, 3- or 5-CHF₂ and 3- or 5-CF₃; and
R¹⁵ is H; or
R¹³ is selected from 3-halogen, 3-(C₁-C₆)alkyl, 3-(C₁-C₆)alkoxy, 3-CH₂F, 3-CHF₂ and 3-CF₃;
R¹⁴ is selected from 5-halogen, 5-(C₁-C₆)alkyl, 5-(C₁-C₆)alkoxy, 5-CH₂F, 5-CHF₂ and 5-CF₃; and
R¹⁵ is H.

4. Salt according to claim 2 or 3, wherein Z¹ is selected from the group consisting of
1-((diphenylmethyl)piperazine;
1-[((4-chlorophenyl)-phenyl-methyl]piperazine;
1-[((4-methoxyphenyl)-phenyl-methyl]piperazine;
1-[((3,5-dichlorophenyl)-phenyl-methyl]piperazine;
1-[((3,4-dichlorophenyl)-phenyl-methyl]piperazine;
1-[((4-fluorophenyl)-phenyl-methyl]piperazine;
1-[((4-methylphenyl)-phenyl-methyl]piperazine;
1-[((4-trifluoromethylphenyl)-phenyl-methyl]piperazine;
(-)-1-[((4-chlorophenyl)-phenyl-methyl]piperazine;
(-)-1-[((4-methoxyphenyl)-phenyl-methyl]piperazine;
(-)-1-[((3,5-dichlorophenyl)-phenyl-methyl]piperazine;
(-)-1-[((3,4-dichlorophenyl)-phenyl-methyl]piperazine;
(-)-1-[((4-fluorophenyl)-phenyl-methyl]piperazine;
(-)-1-[((4-methylphenyl)-phenyl-methyl]piperazine; and
(-)-1-[((4-trifluoromethylphenyl)-phenyl-methyl]piperazine.

5. Salt according to claim 1, which is selected from the group consisting of rosuvastatin 1-[(4-chlorophenyl)-phenyl-methyl]piperazinium salt and rosuvastatin (-)-1-[(4-chlorophenyl)-phenyl-methyl]piperazinium salt.

6. Salt according to claim 5, which is rosuvastatin 1-[(4-chlorophenyl)-phenyl-methyl]piperazinium salt which exhibits a powder x-ray diffraction pattern measured using CuKα-radiation having characteristic peak locations of 10.0, 16.7, 17.9 and 20.2 ± 0.2 degrees 2θ, preferably 10.0, 11.6, 16.7, 17.9, 20.2, 22.5 and 23.7 ± 0.2 degrees 2θ, and more preferably exhibits a powder x-ray diffraction pattern substantially as shown in Fig. 1.

7. Salt according to claim 5, which is rosuvastatin (-)-1-[(4-chlorophenyl)-phenyl-methyl]piperazinium salt which exhibits a powder x-ray diffraction pattern measured using CuKα-radiation having characteristic peak locations of 3.9, 10.0 and 16.9 ± 0.2 degrees 2θ, preferably 3.9, 10.0, 16.5, 16.9, 20.2 and 23.2 ± 0.2 degrees 2θ, and more preferably exhibits a powder x-ray diffraction pattern substantially as shown in Fig. 3.

8. Process for the preparation of a salt according to any one of claims 1 to 7, which process comprises reacting an amine of formula Z with a compound of formula I 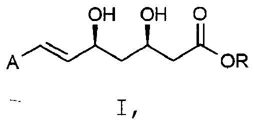 wherein R is selected from the group consisting of H and (C₁-C₁₂)alkyl, or a salt thereof.

9. Process according to claim 8, wherein the amine of formula Z is reacted with a compound of formula I wherein R is (C₁-C₁₂)alkyl, wherein the amine of formula Z is preferably used in an amount of 1 to 5 equivalents.

10. Process according to claim 9, wherein the reaction is carried out in a mixture of organic solvent and water, particularly in a ratio of 10:1 to 1:10, preferably 8:1 to 1:3, more preferably 5:1 to 1:1, most preferably 4:1 to 2:1, wherein the organic solvent is preferably selected from acetonitrile, acetone, methanol, ethanol, isopropanol and mixtures thereof.

11. Process according to claim 10, wherein the reaction is carried out at a temperature of from 0 °C to the boiling point of the solvent mixture, particularly from 10 °C to 70 °C, more preferably 20 °C to 50 °C, most preferably 30 °C to 40 °C.

12. Process for preparing a pharmaceutically acceptable salt of rosuvastatin, preferably rosuvastatin calcium, more preferably rosuvastatin calcium in amorphous or crystalline form, which process comprises converting a salt of formula VII according to any one of claims 1 to 7 to the pharmaceutically acceptable salt of rosuvastatin, wherein the formation of the pharmaceutically acceptable salt is preferably carried out at a pH value of not less than 7.

13. Process according to claim 12, wherein the salt of formula VII is first converted to a corresponding alkaline metal salt, particularly the sodium salt, which is subsequently converted to the pharmaceutically acceptable salt of rosuvastatin.

14. Process according to claim 12 or 13, wherein the formation of the pharmaceutically acceptable salt is carried out in the presence of at least one organic solvent, wherein the at least one organic solvent is preferably used in an amount of 0.0001 to 2 vol%, particularly 0.01 to 1 vol%, more preferably from 0.1 to 0.7 vol%, based on the total volume of the reaction mixture.

15. Process according to claim 14, wherein the reaction is carried out at a temperature of 10 to 40 °C, particularly 15 to 30 °C, more preferably 20 to 25 °C.

16. Process according to claim 14 or 15, wherein the at least one solvent is selected from alcohols, ketones, esters and ethers, and preferably selected from isopropyl acetate and tert-butyl methyl ether.

17. Process according to any one of claims 12 to 16 further comprising re-suspending the pharmaceutically acceptable salt of rosuvastatin in water to obtain a suspension containing 5 to 20 % by weight, preferably 10 % by weight, of said salt and macerating the suspension, wherein maceration is preferably carried out at a temperature of 5 to 25 °C, more preferably 10 to 15 °C.

18. Process according to claim 17, which process comprises
(a) suspending rosuvastatin calcium wet cake in water, to obtain a suspension containing 5 to 20 % by weight, preferably 10% by weight, of rosuvastatin calcium;
(b) stirring the suspension at a temperature of 5 to 25 °C, preferably 10 to 15 °C for 30 min to 2 hours, preferably 30 min to 1 hour, such as about 45 min; and
(c) recovering rosuvastatin calcium by filtration.

## Patentansprüche

1. Salz der Formel VII 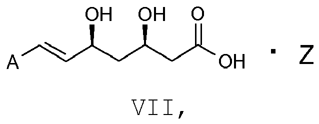 bei dem
A 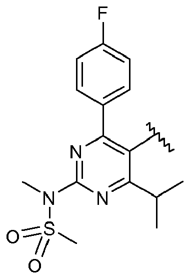 ist und
Z ein 4-(C₁-C₁₂-Alkyl)piperazin ist, bei dem die C₁-C₁₂-AlkylGruppe mindestens einen weiteren Substituenten aufweist, der unabhängig aus der Gruppe bestehend aus unsubstituiertem oder substituiertem Aryl, Heteroaryl, (C₃-C₆)Cycloalkyl und (C₃-C₆)Heterocycloalkyl ausgewählt ist.

2. Salz nach Anspruch 1, bei dem Z ein Amin der Formel Z¹ 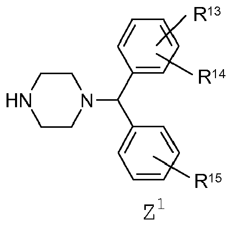 ist, bei dem R¹³, R¹⁴ und R¹⁵ unabhängig aus H, Halogen, (C₁-C₆)Alkyl, (C₁-C₆)Alkoxy, CH₂F, CHF₂ und CF₃ ausgewählt sind, wobei Halogen unabhängig aus F, Cl, Br und I ausgewählt ist.

3. Salz nach Anspruch 2, bei dem
R¹³ aus 4-Halogen, 4-(C₁-C₆)Alkyl, 4-(C₁-C₆)Alkoxy, 4-CH₂F, 4-CHF₂ und 4-CF₃ ausgewählt ist;
R¹⁴ aus H, 3- oder 5-Halogen, 3- oder 5-(C₁-C₆)Alkyl, 3- oder 5-(C₁-C₆)Alkoxy, 3- oder 5-CH₂F, 3- oder 5-CHF₂ und 3- oder 5-CF₃ ausgewählt ist; und
R¹⁵ H ist; oder
R¹³ aus 3-Halogen, 3-(C₁-C₆)Alkyl, 3-(C₁-C₆)Alkoxy, 3-CH₂F, 3-CHF₂ und 3-CF₃ ausgewählt ist;
R¹⁴ aus 5-Halogen, 5-(C₁-C₆)Alkyl, 5-(C₁-C₆)Alkoxy, 5-CH₂F, 5-CHF₂ und 5-CF₃ ausgewählt ist; und
R¹⁵ H ist.

4. Salz nach Anspruch 2 oder 3, bei dem Z¹ aus der Gruppe bestehend aus
1-((Diphenylmethyl)piperazin;
1-[((4-Chlorphenyl)-phenyl-methyl]piperazin;
1-[((4-Methoxyphenyl)-phenyl-methyl]piperazin;
1-[((3,5-Dichlorphenyl)-phenyl-methyl]piperazin;
1-[((3,4-Dichlorphenyl)-phenyl-methyl]piperazin;
1-[((4-Fluorphenyl)-phenyl-methyl]piperazin;
1-[((4-Methylphenyl)-phenyl-methyl]piperazin;
1-[((4-Trifluormethylphenyl)-phenyl-methyl]piperazin;
(-)-1-[((4-Chlorphenyl)-phenyl-methyl]piperazin;
(-)-1-[((4-Methoxyphenyl)-phenyl-methyl]piperazin;
(-)-1-[((3,5-Dichlorphenyl)-phenyl-methyl]piperazin;
(-)-1-[((3,4-Dichlorphenyl)-phenyl-methyl]piperazin;
(-)-1-[((4-Fluorphenyl)-phenyl-methyl]piperazin;
(-)-1-[((4-Methylphenyl)-phenyl-methyl]piperazin; und
(-)-1-[((4-Trifluormethylphenyl)-phenyl-methyl]piperazin
ausgewählt ist.

5. Salz nach Anspruch 1, das aus der Gruppe bestehend aus Rosuvastatin-1-[(4-chlorphenyl)-phenyl-methyl]piperazinium-Salz und Rosuvastatin-(-)-1-[(4-chlorophenyl)-phenyl-methyl]piperazinium-Salz ausgewählt ist.

6. Salz nach Anspruch 5, das Rosuvastatin-1-[(4-chlorphenyl)-phenyl-methyl]piperazinium-Salz ist, welches ein Pulverröntgen beugungsmuster, gemessen mittels CuKα-Strahlung, mit charakteristischen Peak-Positionen von 10,0, 16,7, 17,9 und 20,2 ± 0,2 Grad 2θ, vorzugsweise 10,0, 11,6, 16,7, 17,9, 20,2, 22,5 und 23,7 ± 0,2 Grad 2θ, aufweist, und weiter bevorzugt ein Pulverröntgenbeugungsmuster im Wesentlichen wie in Figur 1 gezeigt aufweist.

7. Salz nach Anspruch 5, das Rosuvastatin (-)-1-[(4-chlorphenyl)-phenyl-methyl]piperazinium-Salz ist, welches ein Pulverröntgenbeugungsmuster, gemessen mittels CuKα-Strahlung, mit charakteristischen Peak-Positionen von 3,9, 10,0 und 16,9 ± 0,2 Grad 2θ, vorzugsweise 3,9, 10,0, 16,5, 16,9, 20,2 und 23,2 ± 0,2 Grad 2θ, aufweist, und weiter bevorzugt ein Pulverröntgenbeugungsmuster im Wesentlichen wie in Figur 3 gezeigt aufweist.

8. Verfahren zur Herstellung eines Salzes gemäß einem der Ansprüche 1 bis 7, bei dem ein Amin der Formel Z mit einer Verbindung der Formel I 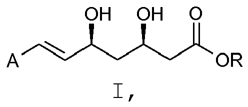 oder einem Salz davon umgesetzt wird, wobei R aus der Gruppe bestehend aus H und (C₁-C₁₂)Alkyl ausgewählt ist.

9. Verfahren nach Anspruch 8, bei dem das Amin der Formel Z mit einer Verbindung der Formel I umgesetzt wird, bei der R (C₁-C₁₂)Alkyl ist, wobei das Amin der Formel Z vorzugsweise in einer Menge von 1 bis 5 Äquivalenten verwendet wird.

10. Verfahren nach Anspruch 9, bei dem die Reaktion in einer Mischung von organischem Lösungsmittel und Wasser, insbesondere in einem Verhältnis von 10:1 bis 1:10, vorzugsweise 8:1 bis 1:3, weiter bevorzugt 5:1 bis 1:1, am meisten bevorzugt 4:1 bis 2:1, durchgeführt wird, wobei das organische Lösungsmittel vorzugsweise aus Acetonitril, Aceton, Methanol, Ethanol, Isopropanol und Mischungen davon ausgewählt ist.

11. Verfahren nach Anspruch 10, bei dem die Reaktion bei einer Temperatur von 0°C bis zum Siedepunkt der Lösungsmittelmischung, insbesondere von 10 °C bis 70 °C, weiter bevorzugt 20 °C bis 50 °C, am meisten bevorzugt 30 °C bis 40 °C, durchgeführt wird.

12. Verfahren zur Herstellung eines pharmazeutisch annehmbaren Salzes von Rosuvastatin, vorzugsweise Rosuvastatin-Calcium, weiter bevorzugt Rosuvastatin-Calcium in amorpher oder kristalliner Form, bei dem ein Salz der Formel VII gemäß einem der Ansprüche 1 bis 7 zu dem pharmazeutisch annehmbaren Salz von Rosuvastatin umgewandelt wird, wobei die Bildung des pharmazeutisch annehmbaren Salzes vorzugsweise bei einem pH-Wert von nicht weniger als 7 durchgeführt wird.

13. Verfahren nach Anspruch 12, bei dem das Salz der Formel VII zunächst zu einem entsprechenden Alkalimetallsalz, vorzugsweise dem Natriumsalz, umgewandelt wird, welches danach zu dem pharmazeutisch annehmbaren Salz von Rosuvastatin umgewandelt wird.

14. Verfahren nach Anspruch 12 oder 13, bei dem die Bildung des pharmazeutisch annehmbaren Salzes in Gegenwart von mindestens einem organischen Lösungsmittel durchgeführt wird, wobei das mindestens eine organische Lösungsmittel vorzugsweise in einer Menge von 0,0001 bis 2 Vol.-%, insbesondere 0,01 bis 1 Vol.-%, weiter bevorzugt 0,1 bis 0,7 Vol.-%, auf Basis des Gesamtvolumens der Reaktionsmischung, verwendet wird.

15. Verfahren nach Anspruch 14, bei dem die Reaktion bei einer Temperatur von 10 bis 40 °C, vorzugsweise 15 bis 30 °C, weiter bevorzugt 20 bis 25 °C, durchgeführt wird.

16. Verfahren nach Anspruch 14 oder 15, bei dem das mindestens eine Lösungsmittel aus Alkoholen, Ketonen, Estern und Ethern ausgewählt ist und vorzugsweise aus Isopropylacetat und tert-Butylmethylether ausgewählt ist.

17. Verfahren nach einem der Ansprüche 12 bis 16, bei dem ferner das pharmazeutisch annehmbare Salz von Rosuvastatin in Wasser resuspendiert wird, um eine Suspension zu erhalten, die 5 bis 20 Gew.-%, vorzugsweise 10 Gew.-%, des Salzes enthält, und die Suspension mazeriert wird, wobei die Mazerierung vorzugsweise bei einer Temperatur von 5 bis 25 °C, weiter bevorzugt 10 bis 15 °C, durchgeführt wird.

18. Verfahren nach Anspruch 17, bei dem
(a) ein feuchter Rosuvastatin-Calcium-Kuchen in Wasser suspendiert wird, um eine Suspension zu erhalten, die 5 bis 20 Gew.-%, vorzugsweise 10 Gew.-%, Rosuvastatin-Calcium enthält;
(b) die Suspension bei einer Temperatur von 5 bis 25 °C, vorzugsweise 10 bis 15 °C, für 30 Minuten bis 2 Stunden, vorzugsweise 30 Minuten bis 1 Stunde, wie etwa 45 Minuten, gerührt wird; und
(c) Rosuvastatin-Calcium durch Filtration gewonnen wird.

## Revendications

1. Sel de formule VII : 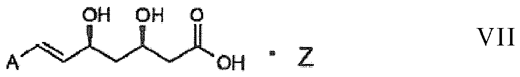 dans laquelle A représente un reste de formule 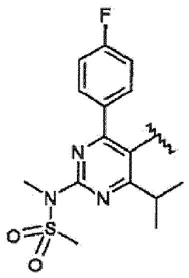 et Z représente une 4-(alkyle en C₁-C₁₂)-pipérazine dont le groupe alkyle en C₁-C₁₂ porte au moins un substituant supplémentaire choisi, indépendamment, dans l'ensemble formé par les groupes aryle, hétéroaryle, cycloalkyle en C₃-C₆ et hétérocycloalkyle en C₃-C₆, avec ou sans substituant(s).

2. Sel conforme à la revendication 1, dans lequel Z représente une amine de formule Z¹ : 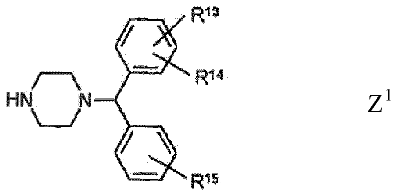 dans laquelle R¹³, R¹⁴ et R¹⁵ représentent des entités choisies, indépendamment, parmi les atomes d'hydrogène et d'halogène, les groupes alkyle en C₁-C₆ et alcoxy en C₁-C₆, et les groupes de formule CH₂F, CHF₂ ou CF₃, étant entendu que les atomes d'halogène sont choisis indépendamment parmi les atomes de fluor, de chlore, de brome et d'iode.

3. Sel conforme à la revendication 2, dans lequel
- R¹³ représente une entité choisie parmi les substituants 4-halogéno, 4-alkyle en C₁-C₆ et 4-alcoxy en C₁-C₆ et les substituants symbolisés par 4-CH₂F, 4-CHF₂ et 4-CF₃,
- R¹⁴ représente une entité choisie parmi un atome d'hydrogène, les substituants 3-halogéno, 5-halogéno, 3-alkyle en C₁-C₆, 5-alkyle en C₁-C₆, 3-alcoxy en C₁-C₆ et 5-alcoxy en C₁-C₆ et les substituants symbolisés par 3-CH₂F, 5-CH₂F, 3-CHF₂, 5-CHF₂, 3-CF₃ et 5-CF₃,
- et R¹⁵ représente un atome d'hydrogène ;
ou bien
- R¹³ représente une entité choisie parmi les substituants 3-halogéno, 3-alkyle en C₁-C₆ et 3-alcoxy en C₁-C₆ et les substituants symbolisés par 3-CH₂F, 3-CHF₂ et 3-CF₃,
- R¹⁴ représente une entité choisie parmi les substituants 5-halogéno, 5-alkyle en C₁-C₆ et 5-alcoxy en C₁-C₆ et les substituants symbolisés par 5-CH₂F, 5-CHF₂ et 5-CF₃,
- et R¹⁵ représente un atome d'hydrogène.

4. Sel conforme à la revendication 2 ou 3, pour lequel l'amine de formule Z¹ est choisie parmi les suivantes :
- 1-(diphényl-méthyl)-pipérazine,
- 1-[(4-chloro-phényl)-phényl-méthyl]-pipérazine,
- 1-[(4-méthoxy-phényl)-phényl-méthyl]-pipérazine,
- 1-[(3,5-dichloro-phényl)-phényl-méthyl]-pipérazine,
- 1-[(3,4-dichloro-phényl)-phényl-méthyl]-pipérazine,
- 1-[(4-fluoro-phényl)-phényl-méthyl]-pipérazine,
- 1-[(4-méthyl-phényl)-phényl-méthyl]-pipérazine,
- 1-[(4-trifluorométhyl-phényl)-phényl-méthyl]-pipérazine,
- (-)-1-[(4-chloro-phényl)-phényl-méthyl]-pipérazine,
- (-)-1-[(4-méthoxy-phényl)-phényl-méthyl]-pipérazine,
- (-)-1-[(3,5-dichloro-phényl)-phényl-méthyl]-pipérazine,
- (-)-1-[(3,4-dichloro-phényl)-phényl-méthyl]-pipérazine,
- (-)-1-[(4-fluoro-phényl)-phényl-méthyl]-pipérazine,
- (-)-1-[(4-méthyl-phényl)-phényl-méthyl]-pipérazine,
- (-)-1-[(4-trifluorométhyl-phényl)-phényl-méthyl]-pipérazine.

5. Sel conforme à la revendication 1, qui est choisi dans l'ensemble formé par un sel de 1-[(4-chloro-phényl)-phényl-méthyl]-pipérazinium de rosuvastatine et un sel de (-)-1-[(4-chloro-phényl)-phénylméthyl]-pipérazinium de rosuvastatine.

6. Sel conforme à la revendication 5, qui est un sel de 1-[(4-chloro-phényl)-phényl-méthyl]-pipérazinium de rosuvastatine qui présente, à l'état de poudre, un diagramme de diffraction des rayons X, mesuré avec la raie Kα du cuivre, dans lequel des pics caractéristiques apparaissent aux angles 2θ de 10,0°, 16,7°, 17,9° et 20,2°, pour chacun à plus ou moins 0,2° près, et de préférence aux angles 2θ de 10,0°, 11,6°, 16,7°, 17,9°, 20,2°, 22,5° et 23,7°, pour chacun à plus ou moins 0,2° près, et mieux encore, présente un diagramme de poudre en diffraction des rayons X qui est pratiquement celui qui est représenté sur la figure 1.

7. Sel conforme à la revendication 5, qui est un sel de (-)-1-[(4-chloro-phényl)-phényl-méthyl]-pipérazinium de rosuvastatine qui présente, à l'état de poudre, un diagramme de diffraction des rayons X, mesuré avec la raie Kα du cuivre, dans lequel des pics caractéristiques apparaissent aux angles 2θ de 3,9°, 10,0° et 16,9°, pour chacun à plus ou moins 0,2° près, et de préférence aux angles 2θ de 3,9°, 10,0°, 16,5°, 16,9°, 20,2° et 23,2°, pour chacun à plus ou moins 0,2° près, et mieux encore, présente un diagramme de poudre en diffraction des rayons X qui est pratiquement celui qui est représenté sur la figure 3.

8. Procédé de préparation d'un sel conforme à l'une des revendications 1 à 7, lequel procédé comporte le fait de faire réagir une amine de formule Z avec un composé de formule I : 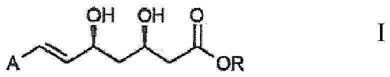 dans laquelle R représente une entité choisie dans l'ensemble formé par un atome d'hydrogène et les groupes alkyle en C₁-C₁₂,
ou avec un sel d'un tel composé.

9. Procédé conforme à la revendication 8, dans lequel on fait réagir une amine de formule Z avec un composé de formule I dans lequel R représente un groupe alkyle en C₁-C₁₂, de préférence en utilisant l'amine de formule Z en une quantité de 1 à 5 équivalents.

10. Procédé conforme à la revendication 9, dans lequel la réaction est réalisée dans un mélange de solvant organique et d'eau, en un rapport valant en particulier de 10/1 à 1/10, de préférence de 8/1 à 1/3, mieux encore de 5/1 à 1/1 et surtout de 4/1 à 2/1, ledit solvant organique étant de préférence choisi parmi l'acétonitrile, l'acétone, le méthanol, l'éthanol et l'isopropanol, ainsi que leurs mélanges.

11. Procédé conforme à la revendication 10, dans lequel la réaction est réalisée à une température valant de 0 °C au point d'ébullition du mélange solvant, en particulier de 10 °C à 70 °C, mieux encore de 20 °C à 50 °C et surtout de 30 °C à 40 °C.

12. Procédé de préparation d'un sel pharmacologiquement admissible de rosuvastatine, de préférence du sel de calcium de rosuvastatine et mieux encore du sel de calcium de rosuvastatine sous forme amorphe ou cristalline, lequel procédé comporte le fait de convertir un sel de formule VII, conforme à l'une des revendications 1 à 7, en un sel pharmacologiquement admissible de rosuvastatine, et dans lequel la formation de ce sel pharmacologiquement admissible est de préférence réalisée à un pH valant au moins 7.

13. Procédé conforme à la revendication 12, dans lequel on convertit d'abord le sel de formule VII en un sel de métal alcalin correspondant, en particulier le sel de sodium, que l'on convertit ensuite en le sel pharmacologiquement admissible de rosuvastatine.

14. Procédé conforme à la revendication 12 ou 13, dans lequel la formation du sel pharmacologiquement admissible est réalisée en présence d'au moins un solvant organique, lequel solvant organique au nombre d'au moins un est de préférence utilisé en un volume représentant de 0,0001 à 2 %, en particulier de 0,01 à 1 % et de préférence de 0,1 à 0,7 % du volume total du mélange réactionnel.

15. Procédé conforme à la revendication 14, dans lequel la réaction est réalisée à une température valant de 10 à 40 °C, en particulier de 15 à 30 °C et mieux encore de 20 à 25 °C.

16. Procédé conforme à la revendication 14 ou 15, dans lequel ledit solvant au nombre d'au moins un est choisi parmi les alcools, cétones, esters et éthers, et choisi de préférence parmi l'acétate d'isopropyle et le tertiobutyl-méthyl-éther.

17. Procédé conforme à l'une des revendications 12 à 16, qui comporte en outre le fait de remettre en suspension dans de l'eau le sel pharmacologiquement admissible de rosuvastatine, de manière à obtenir une suspension contenant de 5 à 20 % en poids et de préférence 10 % en poids dudit sel, et le fait de laisser macérer cette suspension, laquelle macération se déroule de préférence à une température de 5 à 25 °C et de préférence de 10 à 15 °C.

18. Procédé conforme à la revendication 17, lequel procédé comporte les opérations suivantes :
a) mettre en suspension dans de l'eau le gâteau humide de sel de calcium de rosuvastatine, de manière à obtenir une suspension contenant de 5 à 20 % en poids et de préférence 10 % en poids du sel de calcium de rosuvastatine,
b) agiter cette suspension à une température valant de 5 à 25 °C et de préférence de 10 à 15 °C, et ce durant de 30 minutes à 2 heures et de préférence de 30 minutes à 1 heure, comme durant environ 45 minutes,
c) et récupérer par filtration le sel de calcium de rosuvastatine.
